# EUROPEAN PATENT APPLICATION

(11) **EP 4 148 139 A1**
(43) Date of publication of application: **15.03.2023**
(21) Application number: 21825277.3
(22) Date of filing: 21.04.2021
(51) Int. Cl.: C12Q 1/04, C12N 5/071, C12Q 1/68, C12Q 1/6837, G01N 33/48, G01N 33/68

(54) **BIOMARKER IDENTIFICATION METHOD AND CELL PRODUCTION METHOD**

(30) Priority: 19.06.2020 JP 2020106416
(71) Applicant: FUJIFILM Corporation, Tokyo 106-8620 (JP)
(72) Inventor: MURAKAMI, Yuta, Ashigarakami-gun, Kanagawa 258-8577 (JP); NAGASE, Masaya, Ashigarakami-gun, Kanagawa 258-8577 (JP); NOGUCHI, Yukihisa, Ashigarakami-gun, Kanagawa 258-8577 (JP)
(74) Representative: Hoffmann Eitle
(86) International application number: PCT/JP2021/016167
(87) International publication number: WO 2021/256078

(57) **Abstract**

Provided are a biomarker identifying method including (1) to (4) and an application thereof. (1) An evaluation value for each of a plurality of biomarkers is derived based on annotation information imparted to each of biomarkers, and a measurement target biomarker is selected from among the plurality of biomarkers based on the evaluation value. (2) The evaluation data of the measurement target biomarker is acquired from at least one of the cell A or the culture system, at least before the start of culture of the cell A or during the culture. (3) The evaluation data of the discrimination marker of the B cell is acquired from at least one of the cell A or the culture system, at least at the final stage of the culture of the cell A or after the end of the culture. (4) At least one biomarker indicating characteristics of the cell A is identified from among the measurement target biomarkers, based on the evaluation data of the measurement target biomarker and the evaluation data of the discrimination marker of the cell B.

## Description

### BACKGROUND OF THE INVENTION

### 1. Field of the Invention

The technology of the present disclosure relates to a biomarker identifying method and a cell producing method.

### 2. Description of the Related Art

JP2019-020838A discloses a method of constructing a database of gene association information including gene association measurement data that reflects the expression of genes or the function of gene products in a biological specimen.

JP2009-534036B discloses a method of regulating a cell culture phenotype in a cell line or identifying a protein serving as an indicator of the cell culture phenotype.

### SUMMARY OF THE INVENTION

An attempt has been made to identify a characteristic biomarker that constrains the culture efficiency of cells or the quality of cultured cells, for the intended purpose of linking it to the culture step of the cells or the adjustment of the phenotype of the cultured cells. In order to identify the characteristic biomarker, it is conceivable to actually measure all assumable biomarkers; however, it takes time and effort to actually measure all the biomarkers. In addition, even in a case where all assumable biomarkers can be actually measured, it is not always such that there is much knowledge about cell culture in those biomarkers, and the identification of the characteristic biomarker may not be linked to the culture step of the cells or the adjustment of the phenotype of the cultured cells.

Aspects according to the present disclosure have been made under the above circumstances.

An object of the present disclosure is to provide a biomarker identifying method in which a biomarker indicating cell characteristics is rapidly identified and a cell producing method in which a production step is rapidly made to be proper.

Specific means for achieving the object include the following aspects.
<1> A biomarker identifying method that is a method of identifying a biomarker indicating characteristics of a cell A that is used for producing a cell B, the biomarker identifying method comprising the following (1) to (4):
   (1) deriving an evaluation value for each of a plurality of biomarkers based on annotation information imparted to each of the plurality of biomarkers and selecting a measurement target biomarker from among the plurality of biomarkers based on the evaluation value;
   (2) acquiring evaluation data of the measurement target biomarker from at least one of the cell A or a culture system, at least before start of culture of the cell A or during the culture;
   (3) acquiring evaluation data of a discrimination marker of the B cell from at least one of the cell A or the culture system, at least at a final stage of the culture of the cell A or after end of the culture; and
   (4) identifying at least one biomarker indicating characteristics of the cell A that is used for producing the cell B from among the measurement target biomarkers, based on the evaluation data of the measurement target biomarker and the evaluation data of the discrimination marker of the cell B.
<2> The biomarker identifying method according to <1>, in which the biomarker includes at least one selected from the group consisting of an expression level of a gene, an expression level of a protein, and a generation amount of a metabolite.
<3> The biomarker identifying method according to <1> or <2>, in which the (2) is carried out at a plurality of time points.
<4> The biomarker identifying method according to any one of <1> to <3>, in which the cell A is a pluripotent stem cell and the cell B is a differentiated cell.
<5> A cell producing method that is a method of culturing a cell A to produce a cell B, the cell producing method comprising the following (A) to (C):
   (A) carrying out the biomarker identifying method according to any one of <1> to <3> to identify at least one biomarker indicating characteristics of the cell A;
   (B) identifying at least one of a signal transduction in which the biomarker is involved or a mechanism by which the biomarker varies with reference to annotation information of the identified biomarker; and
   (C) culturing the cell A under culture conditions, in which at least one of the signal transduction or the mechanism is inhibited or promoted, to produce the cell B.
<6> The cell producing method according to <5>, in which the cell A is a pluripotent stem cell and the cell B is a differentiated cell.

According to the present disclosure, there are provided a biomarker identifying method in which a biomarker indicating cell characteristics is rapidly identified and a cell producing method in which a production step rapidly made to be proper.

### BRIEF DESCRIPTION OF THE DRAWINGS

Fig. 1 is a diagram illustrating an information processing apparatus.
Fig. 2 is a diagram illustrating gene expression information.
Fig. 3 is a diagram illustrating an annotation information table.
Fig. 4 is a table showing annotation information.
Fig. 5 is a diagram illustrating a situation in which an iPS cell differentiates into three germ layers and then into tissue cells from the three germ layers.
Fig. 6 is a diagram illustrating an outline of processing in the information processing apparatus.
Fig. 7 is a block diagram illustrating a computer that constitutes the information processing apparatus.
Fig. 8 is a block diagram illustrating a processing unit of a CPU of the information processing apparatus.
Fig. 9 is a diagram illustrating a category designation screen and category and number range designation information.
Fig. 10 is a diagram illustrating a state in which a warning screen is subjected to pop-up displaying on the category designation screen.
Fig. 11 is a diagram illustrating an outline of processing in a selection unit.
Fig. 12 is a diagram illustrating a prior known gene designation screen and prior known gene designation information.
Fig. 13 is a diagram illustrating an extraction target designation screen and extraction target designation information.
Fig. 14 is a diagram illustrating a DEGs list.
Fig. 15 is a diagram illustrating distribution information.
Fig. 16 is a diagram illustrating a situation in which an imparted DEGs list is generated in an acquisition unit.
Fig. 17 is a diagram illustrating a situation in which an evaluation value table is generated in a derivation unit.
Fig. 18 is a diagram illustrating a situation in which a prior known gene is unconditionally selected as a measurement target gene in the selection unit.
Fig. 19 is a diagram illustrating a situation in which a selection order table group is generated from the evaluation value table in the selection unit.
Fig. 20 is a diagram illustrating a situation in which DEGs of which the number satisfies a number range are selected in the selection unit and the selected DEGs are allocated as the measurement target gene.
Fig. 21 is a diagram illustrating a measurement target gene list.
Fig. 22 is a diagram illustrating an outline of processing in each of an extraction unit and an acquisition unit.
Fig. 23 is a diagram illustrating an outline of processing in each of a derivation unit and a selection unit.
Fig. 24 is a diagram illustrating a measurement target gene display screen.
Fig. 25 is a flowchart illustrating a processing procedure in the information processing apparatus.
Fig. 26 is a diagram illustrating an example in which annotation information having a relatively high rarity is determined to have a high information value and the number of impartments of the annotation information is increased.
Fig. 27 is a table showing the impartment circumstance of the annotation information for three DEGs.
Fig. 28 is a diagram illustrating a third embodiment in which the weighting of the evaluation value of a gene of which the intensity indicator is within a preset threshold range is increased.
Fig. 29 is a table showing the prior known genes designated for selecting measurement target genes of Examples and extracted DEGs.
Fig. 30 is a diagram illustrating results of the measurement of the gene expression level from a microarray, which is Comparative Example.
Fig. 31 is a table showing high-influence annotation information selected from genes that have become measurement targets in a microarray.
Fig. 32 is a table showing high-influence annotation information selected from genes that have become measurement targets in a microarray.
Fig. 33 is a diagram illustrating measurement results of expression levels of C1000.
Fig. 34 is a table showing high-influence annotation information which has been selected from C1000 and genes to which high-influence annotation information has been imparted.
Fig. 35 is a bar graph of odds ratios from a set of measurement genes of C1000.
Fig. 36 is a bar graph of odds ratios from a set of measurement genes of the TaqMan scorecard of Comparative Example.
Fig. 37 is an example of dot plots showing results obtained by analyzing a cell aggregate obtained by inducing differentiation of an iPS cell into the myocardial cell by flow cytometry.
Fig. 38 is a scatter plot of a prediction model of a cTnT positive rate, created from the regression analysis using a random forest.

### DESCRIPTION OF THE PREFERRED EMBODIMENTS

The term "step" in the present disclosure not only includes, in addition to a step independent of another step, a step that achieves a desired effect of the step even in a case of not being clearly distinguished from the other step.

In the present disclosure, a numerical range described using "to" includes numerical values before and after "to" as a minimum value and a maximum value, respectively.

In the numerical ranges described stepwise in the present disclosure, the upper limit value or the lower limit value described in one numerical range may be replaced with the upper limit value or the lower limit value of the numerical range described stepwise in other stages. Further, in the numerical ranges described in the present disclosure, the upper limit value or the lower limit value of the numerical range may be replaced with the value shown in Examples.

In the present disclosure, each component may contain a plurality of kinds of substances corresponding thereto. In a case where a plurality of kinds of substances corresponding to each component are present in the composition, the content rate or content of each component means the content rate or content of the total of the plurality of kinds of substances present in the composition, unless otherwise specified.

In a case where an embodiment is described in the present disclosure with reference to the accompanying drawing, the configuration of the embodiment is not limited to the configuration shown in the drawing.

In the present disclosure, "X and/or Y" has the same meaning as "at least one of X or Y". That is, it means that "X and/or Y" may be only X, may be only Y, or may be a combination of X and Y In addition, even in a case where three or more matters are connected to described with "and/or" in the present disclosure, the same concept as in "X and/or Y" is applied.

### <Biomarker identifying method>

In the present disclosure, a biomarker means an element that can vary depending on the presence of cells. The biomarker is, for example, an expression level of a gene, an expression level of a protein, a generation amount of a metabolite, a reduction amount of a metabolized chemical substance, a potential of hydrogen (pH) of a culture solution, an O₂ concentration in a culture system, a CO₂ concentration in a culture system, a proportion of live cells, or a proportion of dead cells.

The biomarker identifying method according to the present disclosure is a method of identifying a biomarker indicating characteristics of a cell A that is used for producing a cell B. The biomarker identifying method according to the present disclosure includes the following (1) to (4). The following (1) and the like are also referred to as a step (1) and the like, respectively.
(1) deriving an evaluation value for each of a plurality of biomarkers based on annotation information imparted to each of the plurality of biomarkers and selecting a measurement target biomarker from among the plurality of biomarkers based on the evaluation value;
(2) acquiring evaluation data of the measurement target biomarker from at least one of the cell A or a culture system, at least before start of culture of the cell A or during the culture;
(3) acquiring evaluation data of a discrimination marker of the B cell from at least one of the cell A or the culture system, at least at a final stage of the culture of the cell A or after end of the culture; and
(4) identifying at least one biomarker indicating characteristics of the cell A that is used for producing the cell B from among the measurement target biomarkers, based on the evaluation data of the measurement target biomarker and the evaluation data of the discrimination marker of the cell B.

In the biomarker identifying method according to the present disclosure, a biomarker indicating the characteristics of the cell A that is used for producing the cell B can be rapidly identified by carrying out the steps (1) to (4), and furthermore, the identified biomarker has annotation information, and thus the annotation information can be linked to the culture step of the cell A or the adjustment of the phenotype of the cell B.

The cell A and the cell B may have the same phenotype or may have phenotypes different from each other. The case where the cell A and the cell B have the same phenotype means that the cell A proliferates while maintaining the phenotype thereof, and then the cell B is obtained as a cell that has proliferated. The case where the cell A and the cell B have phenotypes different from each other means that the cell A undergoes a morphological change or cell differentiation, whereby the cell B is obtained.

The cell A and the cell B may be animal cells or may be plant cells. In a case where the cell A is an animal cell, the cell A is derived from, for example, a mammal, and specifically, it is derived from a human; an experimental animal such as a mouse, a rat, a guinea pig, a rabbit, or a monkey; an industrial animal such as a bovine, a horse, a pig, a goat, or a sheep; or a pet animal such as a dog or a cat.

An example of a form of the cell A is a cell capable of differentiating into another cell. In a case where the cell A is an animal cell, the cell A is, for example, a pluripotent stem cell such as an embryonic stem cell (an ES cell) or an induced pluripotent stem cell (an iPS cell); or a multipotent stem cell such as a mesenchymal stem cell, a tissue stem cell, or a somatic stem cell.

The cell A may be a commercially available cell or a distributed cell or may be a cell prepared according to a known method. Examples of the multipotent stem cell include cells derived from any tissue of bone marrow, adipose, blood, synovial membrane, dermis, muscle, umbilical cord, placenta, amnion, chorion, decidua, endometrium, dental follicle, periodontal membrane, dental pulp, or tooth germ. The iPS cell can be prepared from any somatic cell. The somatic cell that is used for producing the iPS cell is not particularly limited. The iPS cell may be produced using a somatic cell in the fetal period, or the iPS cell may be produced using an adult-derived somatic cell (that is, a mature somatic cell). Examples of the somatic cell from which an iPS cell is prepared include (1) a tissue stem cell (or a somatic stem cell) such as a neural stem cell, a hematopoietic stem cell, a mesenchymal stem cell, or a dental pulp stem cell; (2) a tissue progenitor cell; and (3) a differentiated cell such as a fibroblast (a skin cell or the like), an epithelial cell, a hepatocyte, a lymphocyte (for example, T cell or B cell), an endothelial cell, a muscle cell, a hair cell, a gastric mucosal cell, an intestinal cell, a splenocyte, a pancreatic cell (an exocrine pancreatic cell or the like), a brain cell, a lung cell, a kidney cell, and a skin cell.

An example of a form of the cell B is a cell that has differentiated from a cell having differentiation potency. In a case where the cell B is an animal cell, the cell B may be any somatic cell.

Examples of the cell B include endodermal cells such as a cholangiocyte (a hepatocyte, hepatic sinusoidal endothelial cell, a Kupper cell, a hepatic stellate cell, a pit cell, a ductal cell, a mesothelial cell, a pancreatic endocrine cell, an acinar cell, a duct cell, an absorptive cell, a goblet cell, a Paneth cell, an intestinal endocrine cell, or the like) and a cell of a tissue such as liver or thyroid gland; mesodermal cells such as a blood cell/lymphoid cell (a hematopoietic stem cell, an erythrocyte, a platelet, a macrophage, a granulocyte, a helper T cell, a killer T cell, a B cell, or the like), a vascular system cell (a vascular endothelial cell or the like), a myocardial cell (an atrial cardiac muscle cell, a ventricular cardiac muscle cell, or the like), an osteoblast, a bone cell, a cartilage cell, a tendon cell, an adipose cell, a skeletal muscle cell, and a smooth muscle cell; ectodermal cells such as a neural cell, a sensory organ cell (a cell of crystalline lens, retina, inner ear, or the like), an epidermal cell, and a hair follicle cell.

In an example of the embodiment of the present disclosure, the cell A is a pluripotent stem cell such as an ES cell or an iPS cell, and the cell B is a differentiated cell derived from the pluripotent stem cell.

### [Step (1)]

In the step (1), an evaluation value for each of a plurality of biomarkers is derived based on annotation information imparted to each of the plurality of biomarkers, and a measurement target biomarker is selected from among the plurality of biomarkers based on the evaluation value.

The kind of the measurement target biomarker is not limited, and examples thereof include all kinds of biomarkers. The measurement target biomarker is preferably an expression level of a gene from the viewpoint that a plurality of biomarkers can be measured at one time. An expression level of a gene is preferably measured by extracting the total RNA from the cell A.

The expression level of the gene can be measured by using reverse transcription polymerase chain reaction (RT-PCR), a microarray, RNA-Seq analysis using a next-generation sequencer, or the like.

An expression level of a protein can be measured by enzyme-linked immunosorbent assay (ELISA), flow cytometry, liquid chromatography-tandem mass spectrometry (LC-MS/MS), or the like. The expression level of the protein can also be calculated by labeling the protein by an immunological method, acquiring a labeled image on a computer, and carrying out image analysis.

The generation amount of the metabolite or the reduction amount of the metabolized chemical substance can be determined by measuring the metabolite or the amount of the metabolized chemical substance in the culture medium using LC-MS/MS or a bioanalyzer equipped with an enzyme membrane biosensor.

Hereinafter, as an example of the embodiment of the step (1), an information processing apparatus that realizes the step (1) and an operation method of the information processing apparatus will be illustrated. It is noted that the embodiment of the step (1) is not limited to the following example.

The information processing apparatus includes at least one processor, the processor acquires annotation information imparted to each of a plurality of biomarkers related to a biological specimen, an evaluation value for each of a plurality of biomarkers is derived based on the annotation information, and a measurement target biomarker is selected from among the plurality of biomarkers based on the evaluation value. A specific example of the "biological specimen" is a "cell".

In the operation method of the information processing apparatus, a processor executes an acquisition processing of acquiring annotation information imparted to each of a plurality of biomarkers related to a biological specimen, a derivation processing of deriving an evaluation value for each of a plurality of biomarkers based on the annotation information, and a selection processing of selecting a measurement target biomarker from among the plurality of biomarkers based on the evaluation value.

It is preferable that the processor selects annotation information related to the characteristics of the biological specimen of interest (for example, the characteristics of the cell of interest) and derives an evaluation value based only on the selected annotation information.

It is preferable that the processor imparts annotation information to the biomarker with reference to a database in which the annotation information for the biomarker is registered.

It is preferable that the annotation information is associated with the kind of the biological specimen (for example, the kind of the cell).

It is preferable that the processor receives, the designation by a user for a plurality of categories defined according to the kind of the biological specimen (for example, the kind of the cell) and for a range of the number of measurement target biomarkers for each of the plurality of categories, selects biomarkers, the numbers of which satisfies the range, from the biomarkers prepared for each of the plurality of categories, and allocates the selected biomarkers to each of the plurality of categories as the measurement target biomarker.

The category preferably includes the iPS cell, the ectoderm, the mesoderm, and the endoderm.

It is preferable that the processor counts the number of impartments of the annotation information for each of the plurality of biomarkers and derives an evaluation value based on the number of impartments.

It is preferable that the processor carries out weighting of the evaluation value according to the information value of the annotation information.

It is preferable that the processor determines that the annotation information having a relatively high rarity has a high information value and increases the weighting of the evaluation value.

It is preferable that the processor carries out weighting of the evaluation value based on the orthogonality of the annotation information.

It is preferable that the processor increases the weighting of the evaluation value of the biomarker of which the intensity indicator is within a preset threshold range.

It is preferable that the processor receives the designation by a user for the prior known marker, which is a biomarker that is already known to affect the characteristics (for example, the characteristics of the cell) of the biological specimen and increases the weighting of the evaluation value of the prior known marker.

It is preferable that the processor selects more than 100 and 1,000 or less of measurement target biomarkers.

The biomarker preferably includes a gene.

It is preferable that the gene includes differentially expressed genes (DEGs) in which the expression level specifically varies.

The annotation information is preferably a term defined in the gene ontology.

The information processing apparatus and the operation method of the information processing apparatus will be described in more detail with reference to the drawings. In the following description, a form in which a user of the technology of the present disclosure searches for an expression level of a gene as a biomarker will be described as an example in a case of inducing the cell A to differentiate to produce the cell B.

### [First embodiment]

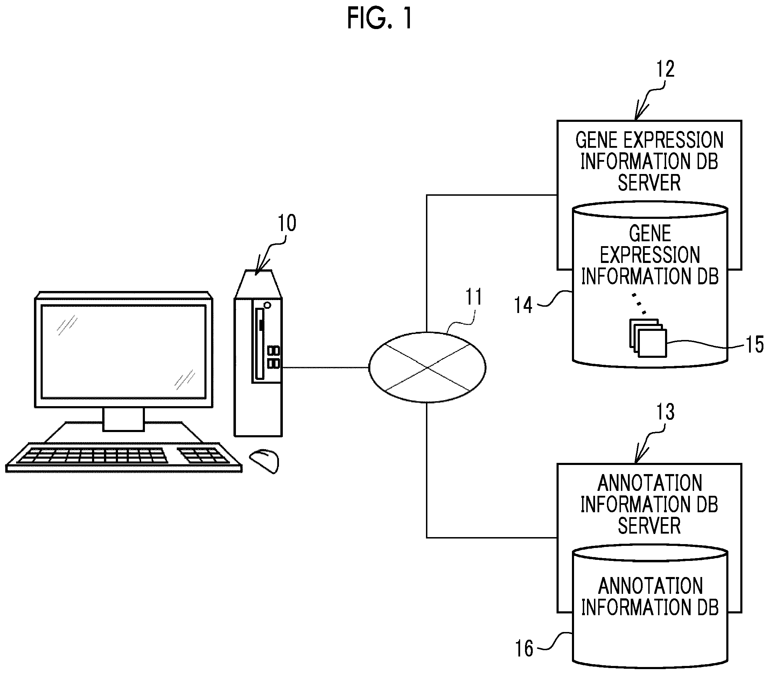

In Fig. 1, an information processing apparatus 10 is, for example, a desktop personal computer, and it is operated by a user. The information processing apparatus 10 is connected to a network 11. The network 11 is, for example, the Internet or a wide area network (WAN) such as a public telecommunication network.

The information processing apparatus 10 is connected to a gene expression information DB server 12 (DB is an abbreviation for a database) and an annotation information DB server 13 via the network 11. The gene expression information DB server 12 has a gene expression information DB 14. The gene expression information DB 14 is, for example, Gene Expression Omnibus (GEO) provided by the National Center for Biotechnology Information (NCBI). A huge amount of gene expression information 15 uploaded from an unspecified number of researchers is registered as open data in the gene expression information DB 14. Gene expression information 15 is information related to an expression level of a gene expressed by a cell during culture.

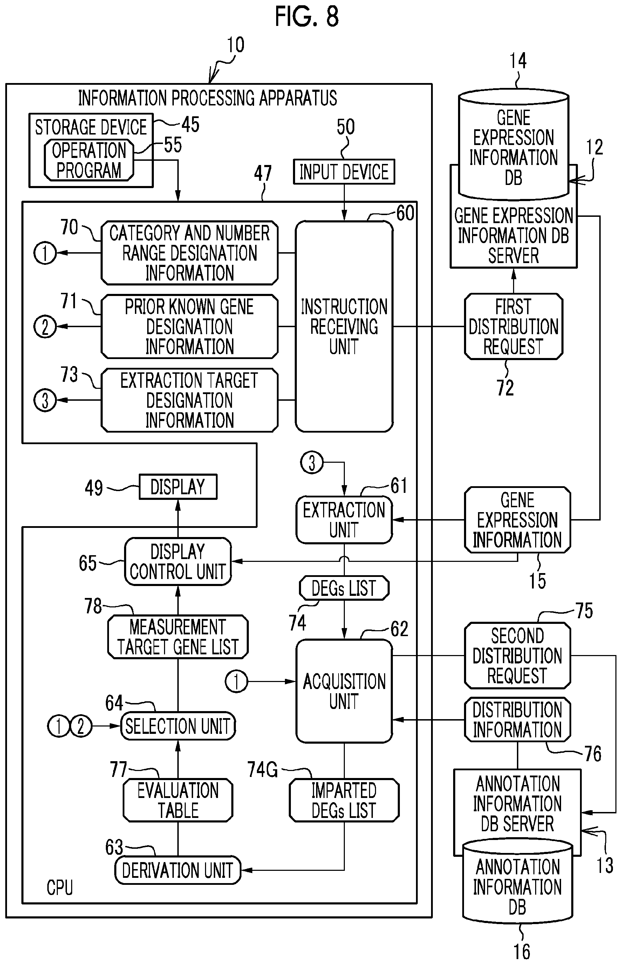

The gene expression information DB server 12 receives a first distribution request 72 (see Fig. 8) from the information processing apparatus 10. The gene expression information DB server 12 reads out the gene expression information 15 in response to the first distribution request 72, from the gene expression information DB 14. Then, the read-out gene expression information 15 is distributed to the information processing apparatus 10.

The annotation information DB server 13 has an annotation information DB 16. The annotation information DB 16 is, for example, the Database for Annotation, Visualization and Integrated Discovery (DAVID) provided by the National Institute of Allergy and Infectious Diseases (NIAID), and/or InterPro provided by the European Bioinformatics Institute (EBI). Corresponding annotation information is registered in the annotation information DB 16 for each of the plurality of genes.

The annotation information DB server 13 receives a second distribution request 75 (see Fig. 8) from the information processing apparatus 10. The annotation information DB server 13 reads out the annotation information in response to the second distribution request 75 from the annotation information DB 16. Then, the distribution information 76 (see Fig. 8) including the read-out annotation information is distributed to the information processing apparatus 10.

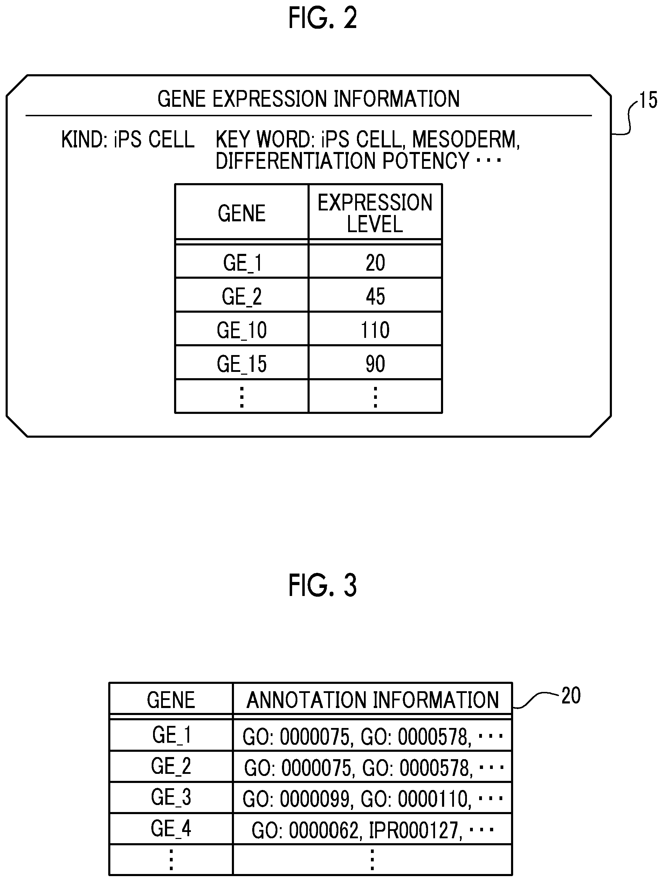

As illustrated in Fig. 2, the gene expression information 15 is information in which an expression level is registered for each gene. In the gene expression information 15, a kind ("iPS cell" in Fig. 2) of a biological specimen of which the expression level has been measured is registered. In addition, in the gene expression information 15, keywords such as "iPS cell", "mesoderm", and "differentiation potency" are registered for facilitating a search. The keyword is registered, for example, by a researcher who has uploaded the gene expression information 15 or a provider of the gene expression information DB 14.

An annotation information table 20 illustrated in Fig. 3 is stored in the annotation information DB 16. In the annotation information table 20, annotation information (ID; Identification Data) is registered for each gene.

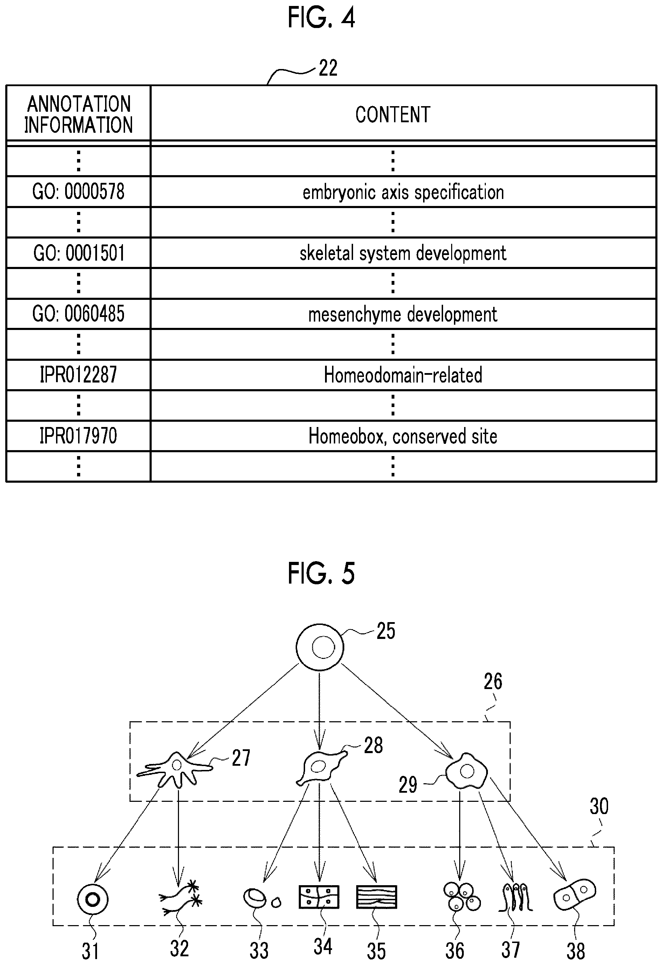

As shown in Table 22 of Fig. 4, the annotation information is a term defined by Gene Ontology (GO), such as "embryonic axis specification" of ID "GO:0000578" or "Homeodomain-related" of ID "IPR012287".

As illustrated in Fig. 5, an exemplary case where an iPS cell 25 established by reprogramming a human somatic cell is used as a research target is described as below. The iPS cell 25 forms three germ layers 26 by cell division. The three germ layers 26 are an ectoderm 27, a mesoderm 28, and an endoderm 29. Each of the three germ layers 26 differentiates into a plurality of kinds of tissue cells 30. Specifically, the ectoderm 27 differentiates into a crystalline lens 31, a nerve cell 32, and the like. The mesoderm 28 differentiates into a blood cell 33, a bone cell 34, a muscle cell 35, and the like. The endoderm 29 differentiates into an alveolar cell 36, an intestinal cell 37, a hepatocyte 38, and the like.

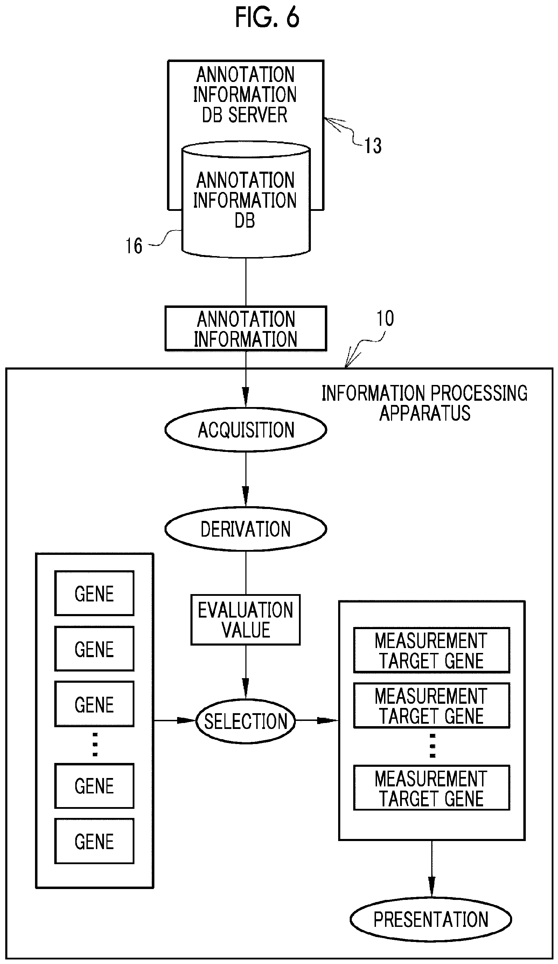

Fig. 6 illustrates an outline of processing of the information processing apparatus 10. First, the information processing apparatus 10 acquires the annotation information from the annotation information DB server 13. Then, an evaluation value for each gene is derived based on the acquired annotation information. Next, a gene to be measured (hereinafter, referred to as a measurement target gene) is selected from among a plurality of genes based on the derived evaluation value. At this time, the information processing apparatus 10 selects measurement target genes, the number of which has been designated by a user. The number of genes that are candidates for the measurement target gene is, for example, about 3,000, and the number of measurement target genes is, for example, 1,000. The information processing apparatus 10 presents the selected measurement target gene to a user.

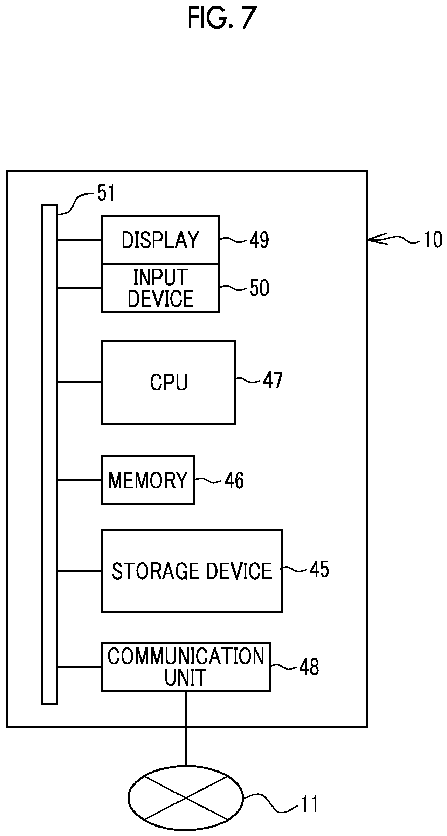

In Fig. 7, the computer that constitutes the information processing apparatus 10 includes a storage device 45, a memory 46, a central processing unit (CPU) 47, a communication unit 48, a display 49, and an input device 50. These components are connected to each other through a bus line 51.

The storage device 45 is a hard disk drive that is built in the computer that constitutes the information processing apparatus 10 or is connected to the computer through a cable or a network. Alternatively, the storage device 45 is a disk array in which a plurality of hard disk drives are connectively mounted. The storage device 45 stores a control program such as an operating system, various application programs, various types of data associated with these programs, and the like. A solid state drive may be used instead of the hard disk drive.

The memory 46 is a work memory that is used in a case where the CPU 47 executes processing. The CPU 47 loads the program stored in the storage device 45 into the memory 46 and executes processing according to the program, thereby comprehensively controlling each of the units of the computer.

The communication unit 48 is a network interface that controls transmission of various types of information via the network 11. The display 49 displays various screens. The computer that constitutes the information processing apparatus 10 receives an input of an operation instruction from the input device 50, through the various screens. The input device 50 is a keyboard, a mouse, a touch panel, or the like.

In Fig. 8, an operation program 55 is stored in the storage device 45 of the information processing apparatus 10. The operation program 55 is an application program for making a computer function as the information processing apparatus 10.

In a case where the operation program 55 is started, the CPU 47 of the computer, which constitutes the information processing apparatus 10, cooperates with the memory 46 and the like to function as an instruction receiving unit 60, an extraction unit 61, an acquisition unit 62, a derivation unit 63, a selection unit 64, and a display control unit 65. The CPU 47 is an example of the "processor".

The instruction receiving unit 60 receives various instructions by a user via the input device 50. For example, the instruction receiving unit 60 receives the designation by a user for a plurality of categories as well as a range of the number of measurement target genes (hereinafter, referred to as a number range) for each of the plurality of categories. The category is defined by a user according to the kind of the biological specimen (for example, the kind of the cell). The instruction receiving unit 60 generates category and number range designation information 70 in response to the designated category and the number range and outputs the category and number range designation information 70 to the selection unit 64.

The instruction receiving unit 60 also receives the designation by a user for the prior known gene. The instruction receiving unit 60 generates prior known gene designation information 71 in response to the designated prior known gene and outputs the prior known gene designation information 71 to the selection unit 64. The prior known gene is a gene that is already known to affect the behavior of the iPS cell 25. That is, the prior known gene is an example of the "prior known marker". The behavior of the iPS cell 25 is an example of "the characteristics of the biological specimen" according to the technology of the present disclosure.

The instruction receiving unit 60 also receives a first distribution instruction by a user, which instructs the gene expression information DB server 12 to distribute the gene expression information 15. Specifically, the first distribution instruction is a search instruction composed of search keywords related to the iPS cell 25, for example, "iPS cell", "ectoderm", "mesoderm", "endoderm", and the like. The first distribution instruction is given through a search screen (not illustrated in the drawing) on which an input box for a search keyword and a search button are provided. In a case where the instruction receiving unit 60 receives the first distribution instruction, it transmits the first distribution request 72 including the search keyword to the gene expression information DB server 12. From among the gene expression information 15 present in the gene expression information DB 14, the gene expression information DB server 12 searches the gene expression information 15 in which the registered keyword matches with the search keyword. Then, the searched gene expression information 15 is distributed to the information processing apparatus 10. In the information processing apparatus 10, the gene expression information 15 is input to the extraction unit 61 and the display control unit 65.

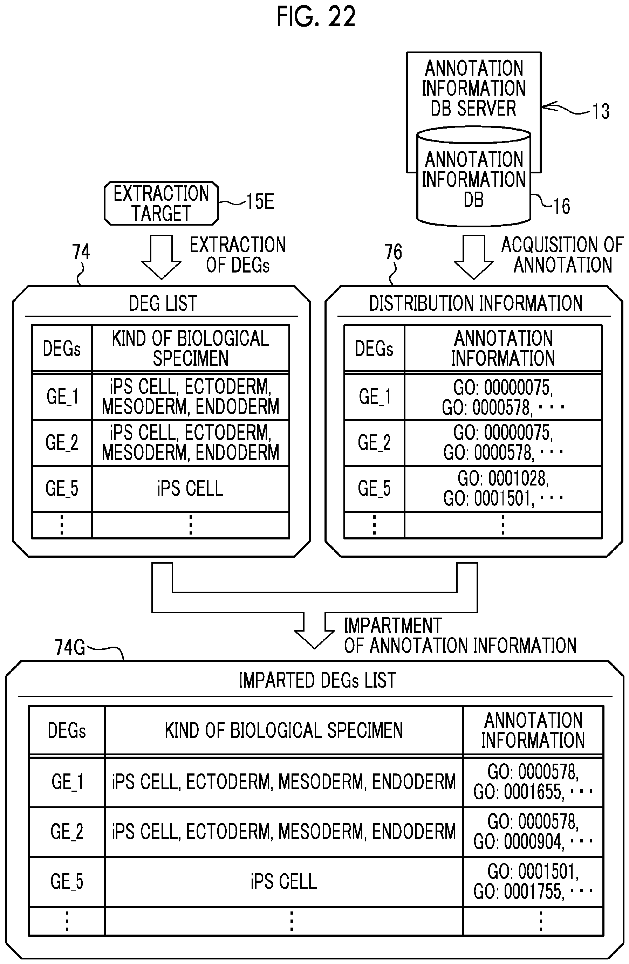

The display control unit 65 displays a display screen (not illustrated in the drawing) of the gene expression information 15 from the gene expression information DB server 12, on the display 49. The instruction receiving unit 60 receives, the designation by a user for the gene expression information 15 (hereinafter, referred to as an extraction target 15E (see Fig. 22)) as a target from which the DEGs are to be extracted, among the displayed gene expression information 15. The instruction receiving unit 60 generates extraction target designation information 73 in response to the designated extraction target 15E and outputs the extraction target designation information 73 to the extraction unit 61.

The extraction unit 61 extracts the DEGs from the extraction target 15E designated by the extraction target designation information 73. For example, the extraction unit 61 compares the expression level of each gene of the extraction target 15E with a preset threshold value and extracts, as DEGs, genes of which the expression level is equal to or higher than the threshold value. The extraction unit 61 generates a DEGs list 74 in which the extracted DEGs are registered and outputs the DEGs list 74 to the acquisition unit 62.

The acquisition unit 62 transmits the second distribution request 75 based on the DEGs list 74 from the extraction unit 61 to the annotation information DB server 13. The second distribution request 75 includes the DEGs registered in the DEGs list 74. The annotation information DB server 13 searches for the annotation information imparted to the DEGs included in the second distribution request 75, from among the annotation information table 20 present in the annotation information DB 16. Then, the distribution information 76, which is composed of the searched annotation information and the set of the DEGs, is distributed to the information processing apparatus 10. In the information processing apparatus 10, the distribution information 76 is input to the acquisition unit 62.

The acquisition unit 62 acquires the distribution information 76 from the annotation information DB server 13. The distribution information 76 includes annotation information as described above. As a result, the acquisition unit 62 acquires the annotation information by acquiring the distribution information 76.

Based on the distribution information 76, the acquisition unit 62 imparts the annotation information to the DEGs list 74 and sets the DEGs list 74 as the imparted DEGs list 74G. That is, the acquisition unit 62 imparts the annotation information to the gene with reference to the annotation information DB 16. The acquisition unit 62 outputs the imparted DEGs list 74G to the derivation unit 63.

The derivation unit 63 derives an evaluation value for each of the DEGs based on the imparted DEGs list 74G. Then, an evaluation value table 77, which is the results of deriving the evaluation values, is output to the selection unit 64.

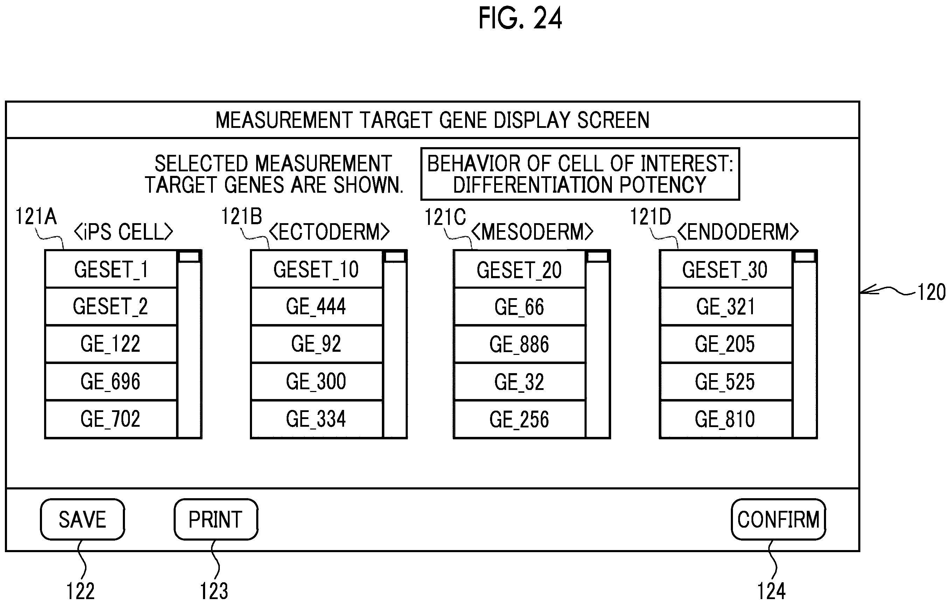

The selection unit 64 unconditionally selects the prior known gene as the measurement target gene according to the prior known gene designation information 71. In addition, the selection unit 64 selects the measurement target gene from among the DEGs extracted by the extraction unit 61 according to the category and number range designation information 70. The selection unit 64 outputs a measurement target gene list 78, which is the selection results of the measurement target gene, to the display control unit 65. The display control unit 65 generates a measurement target gene display screen 120 (see Fig. 24) based on the measurement target gene list 78 and displays this on the display 49.

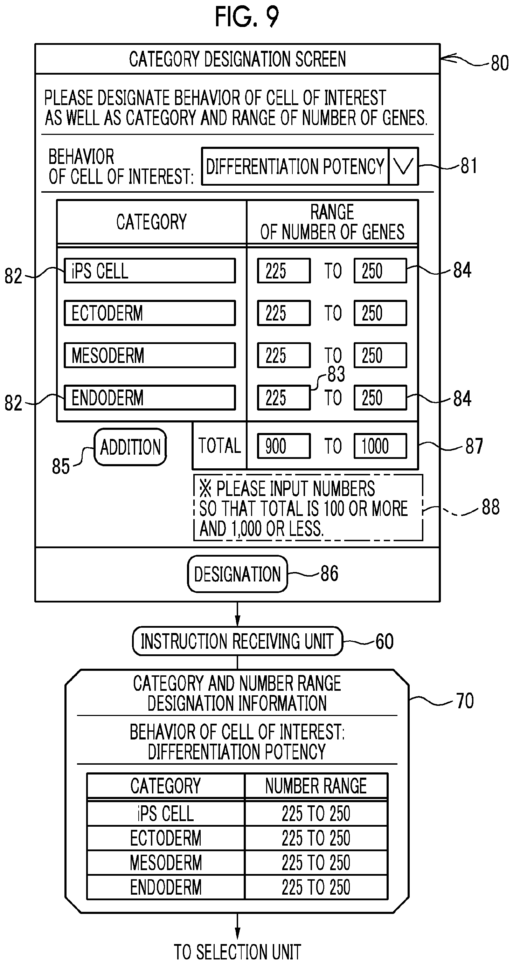

In Fig. 9, in order to receive the designation by a user for the category and the number range, a category designation screen 80 is displayed on the display 49 under the control of the display control unit 65. A pull-down menu 81 is provided on the category designation screen 80, where the pull-down menu 81 is for selectively inputting the behavior of the cell of interest, which is an example of "the characteristics of the biological specimen of interest". Further, an input box 82 of the category, an input box 83 for the lower limit of the number range, and an input box 84 for the upper limit thereof are provided on the category designation screen 80. The input boxes 82 to 84 can be added by selecting an add button 85.

In a case where the behavior of the cell of interest is selected in the pull-down menu 81, a desired category and number range are input in the input boxes 82 to 84, and then the designation button 86 is selected, the instruction receiving unit 60 receives the designation for the behavior of the cell of interest, the category, and the number range. As a result, the category and number range designation information 70 is output from the instruction receiving unit 60 to the selection unit 64. The category and number range designation information 70 includes the behavior of the cell of interest selected in the pull-down menu 81, the category input in the input box 82, and the number range input in the input boxes 83 and 84.

Fig. 9 illustrates an exemplary case where "differentiation potency" is selected as the behavior of the cell of interest. Further, it illustrates an exemplary case where "iPS cell", "ectoderm", "mesoderm", and "endoderm" are designated as the categories, and "225 to 250" is designated for each category as the number range. Only one category may be designated. Further, the same numerical value may be input to the input boxes 83 and 84.

A display region 87 is provided in the lower part of the input boxes 83 and 84, where the display region 87 is for displaying the total of the lower limit and the upper limit of the number range, which are respectively input to the input boxes 83 and 84. A message 88 that urges a user to set the total number to be more than 100 and 1,000 or less is displayed in the lower part of the display region 87.

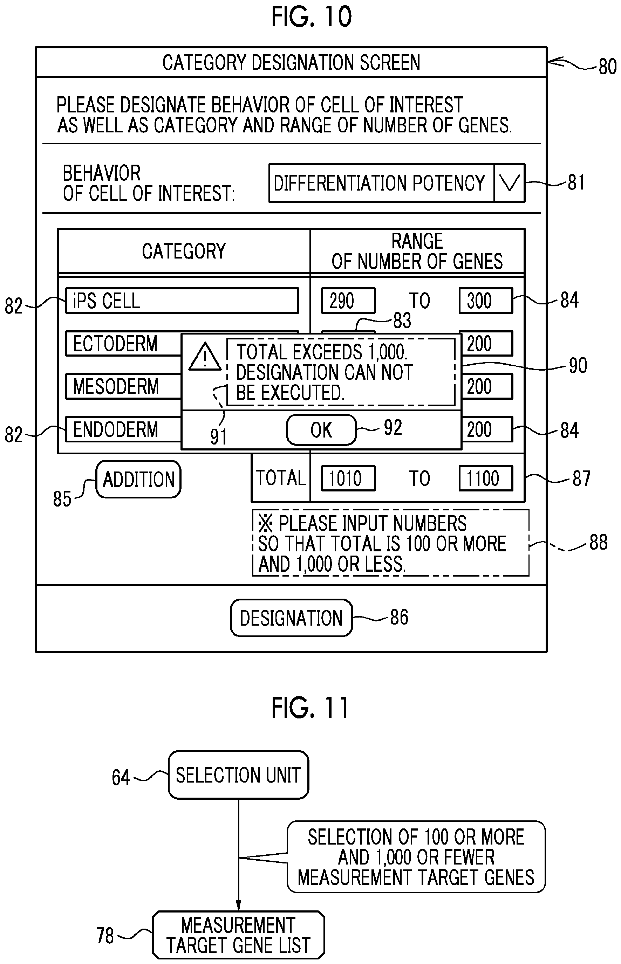

As illustrated in Fig. 10, in a case where the designation button 86 is selected in a state where the total number is out of the range of more than 100 and 1,000 or less, the display control unit 65 causes the warning screen 90 to be subjected to pop-up displaying on the category designation screen 80. A message 91, indicating that the total number is out of the range of more than 100 and 1,000 or less and thus the designation cannot be carried out as it is, is displayed on the warning screen 90. In a case where an OK button 92 is selected, the display control unit 65 turns off the display of the warning screen 90.

The category designation screen 80 is configured such that it cannot be designated in a case where the total of the number range is out of the range of more than 100 and 1,000 or less. As a result of this reason, as illustrated in Fig. 11, the selection unit 64 selects more than 100 and 1,000 or fewer measurement target genes.

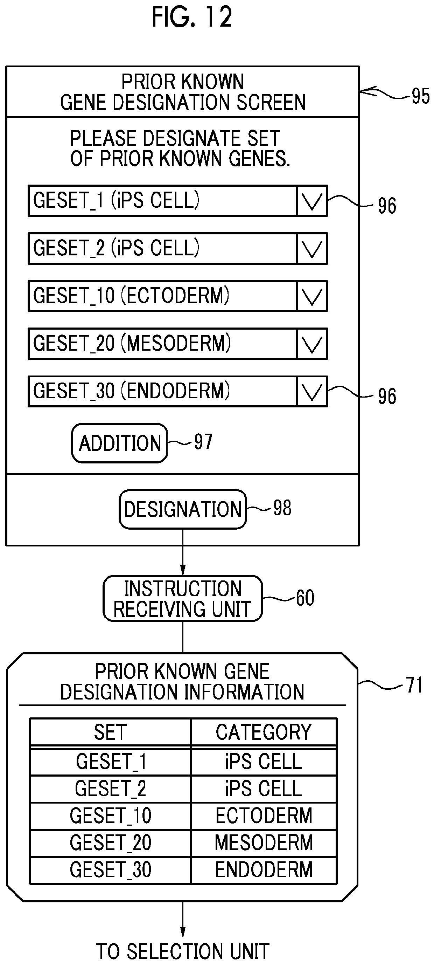

In Fig. 12, the prior known gene designation screen 95 is displayed on the display 49 under the control of the display control unit 65 in order to receive the designation by a user for the prior known gene. A pull-down menu 96 for selectively inputting a set of the prior known genes is provided on the prior known gene designation screen 95. The pull-down menu 96 can be added by selecting an add button 97. In the pull-down menu 96, a plurality of sets of the prior known genes is prepared in advance as an option. The set of the prior known genes is prepared for each category. Examples of the set of the prior known genes include a set of prior known genes that are used for gene analysis using TaqMan (registered trade name) Scorecard, a set of prior known genes that are used for gene analysis using nCounter (registered trade name), and a set of prior known genes that are used for gene analysis using TruSeq (registered trade name).

In a case where a desired set of prior known genes is selected in the pull-down menu 96, and then the designation button 98 is selected, the instruction receiving unit 60 receives the designation for the set of the prior known genes. As a result, the prior known gene designation information 71 is output from the instruction receiving unit 60 to the selection unit 64. The prior known gene designation information 71 is information in which the set of the prior known genes and the category corresponding thereto are registered.

Fig. 12 illustrates an exemplary case where sets of five prior known genes are designated in total is illustrated, where the five sets are two sets of prior known genes for the category "iPS cell" and each one set of prior known genes for each of the categories "ectoderm", "mesoderm", and "endoderm". Instead of the designation of the set or in addition to the designation of the set, the prior known gene may be designated one by one.

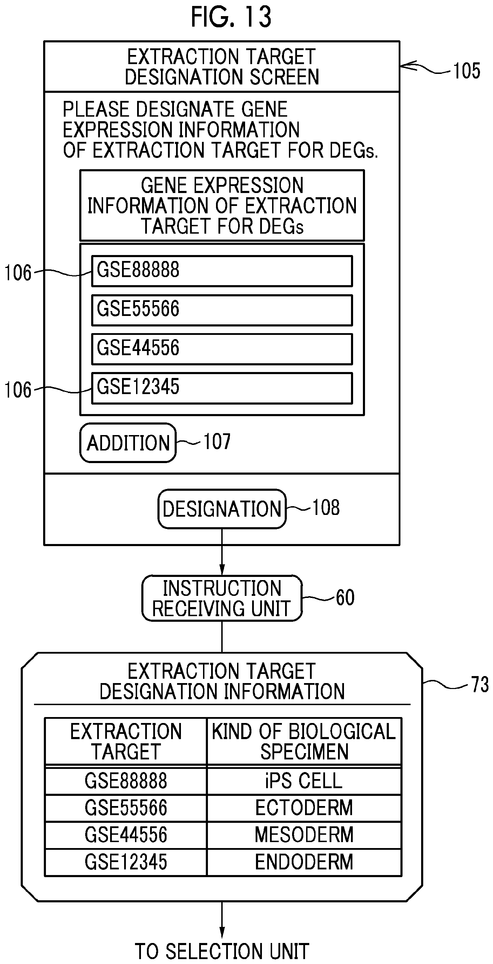

In Fig. 13, the extraction target designation screen 105 is displayed on the display 49 under the control of the display control unit 65 in order to cause a user to designate the extraction target 15E. An input box 106 for the extraction target 15E is provided on the extraction target designation screen 105. The input box 106 can be added by selecting an add button 107.

In a case where the extraction target 15E is input to the input box 106, and then the designation button 108 is selected, the instruction receiving unit 60 receives the designation for the extraction target 15E. As a result, the extraction target designation information 73 is output from the instruction receiving unit 60 to the extraction unit 61. The extraction target designation information 73 is information in which the extraction target 15E input to the input box 106 and the kind of the biological specimen (for example, the kind of the cell) registered in the extraction target 15E are registered.

Fig. 13 illustrates an exemplary case where the extraction target 15E is designated one by one for each of "iPS cell", "ectoderm", "mesoderm", and "endoderm". Two or more extraction targets 15E may be designated for one kind of biological specimen.

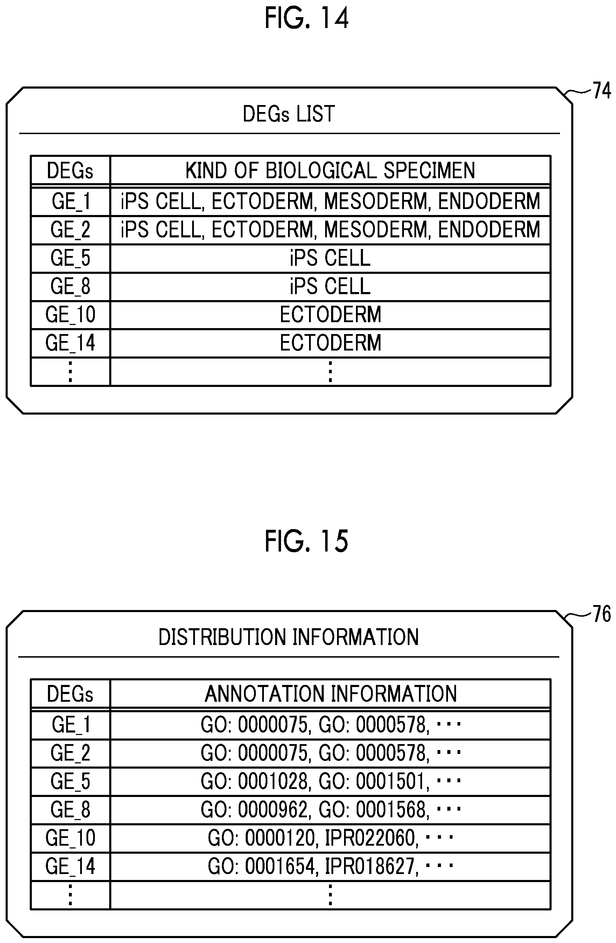

As illustrated in Fig. 14, in the DEGs list 74, the DEGs and the kinds of biological specimens registered in the extraction target 15E from which the DEGs have been extracted are registered. In some DEGs, only one kind of biological specimen is registered as in DEGs having IDs "GE_5", "GE_10", and the like, and in other DEGs, a plurality kind of biological specimens such as "iPS cell", "ectoderm", "mesoderm", and "endoderm" are registered as in DEGs having IDs "GE_1", "GE_2", and the like. That is, some DEGs belong to only one kind of biological specimen, and other DEGs belong to a plurality of kinds of biological specimens.

As illustrated in Fig. 15, the distribution information 76 is information in which the DEGs and the annotation information corresponding thereto are registered.

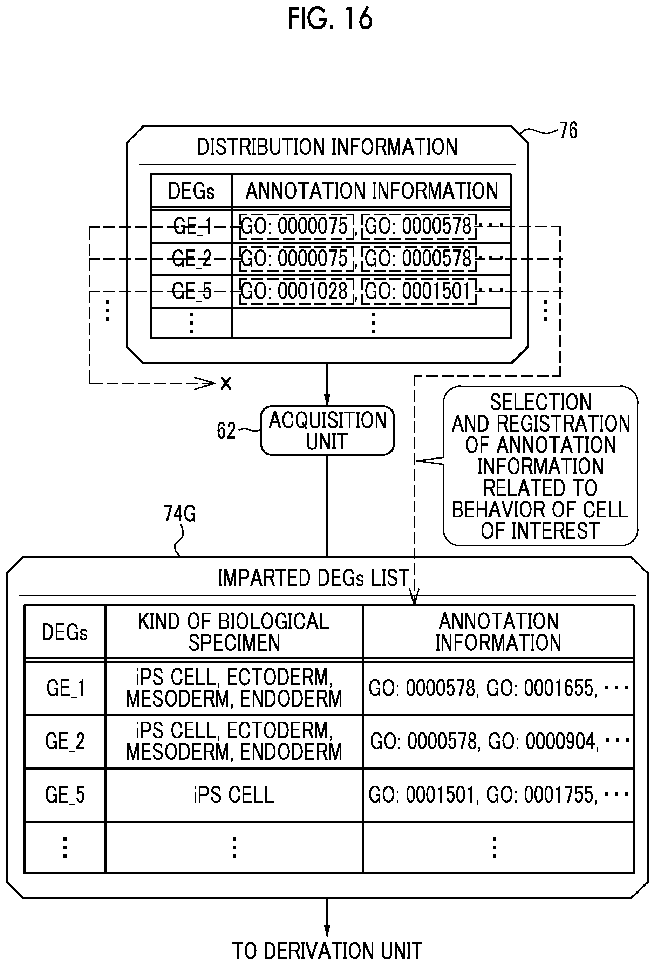

In Fig. 16, the imparted DEGs list 74G is obtained by adding an item of annotation information to the DEGs list 74 illustrated in Fig. 14. By the imparted DEGs list 74G, the kind of the biological specimen (for example, the kind of the cell) is associated with the annotation information.

The acquisition unit 62 selects the annotation information related to the behavior of the cell of interest of the category and number range designation information 70 from among the annotation information registered in the distribution information 76. Then, only the selected annotation information is registered in the DEGs list 74 and is set as the imparted DEGs list 74G.

As illustrated in Fig. 9, in this example, "differentiation potency" is designated as the behavior of the cell of interest. Therefore, the acquisition unit 62 does not select the annotation information having no relation to the differentiation potency, such as IDs "GO: 0000075" and "GO: 0001028", but it selects and registers only the annotation information related to the differentiation, such as IDs "GO: 0000578" and "GO: 0001501". A search keyword related to the behavior of the cell of interest may be included in the second distribution request 75, and the annotation information related to the behavior of the cell of interest may be selected in the annotation information DB server 13.

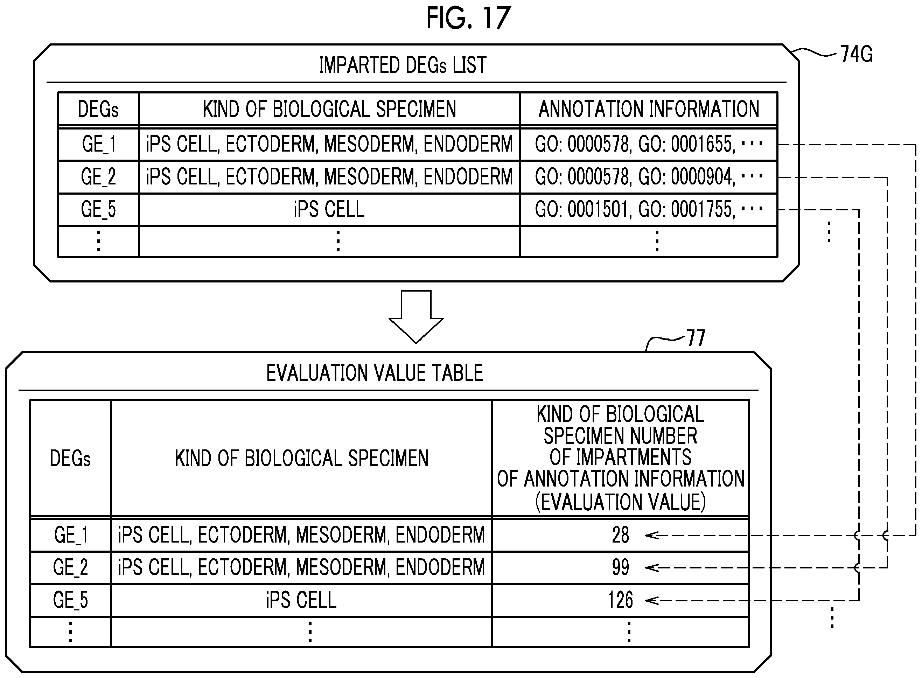

In Fig. 17, the derivation unit 63 counts the number of impartments of the annotation information imparted to each of the DEGs based on the imparted DEGs list 74G. Then, the counted number of impartments itself is registered in the evaluation value table 77 as an evaluation value. For example, in a case where 28 pieces of annotation information are imparted to the DEGs of ID "GE_1", "28" which is the same as the number of impartments is registered as an evaluation value in the evaluation value table 77.

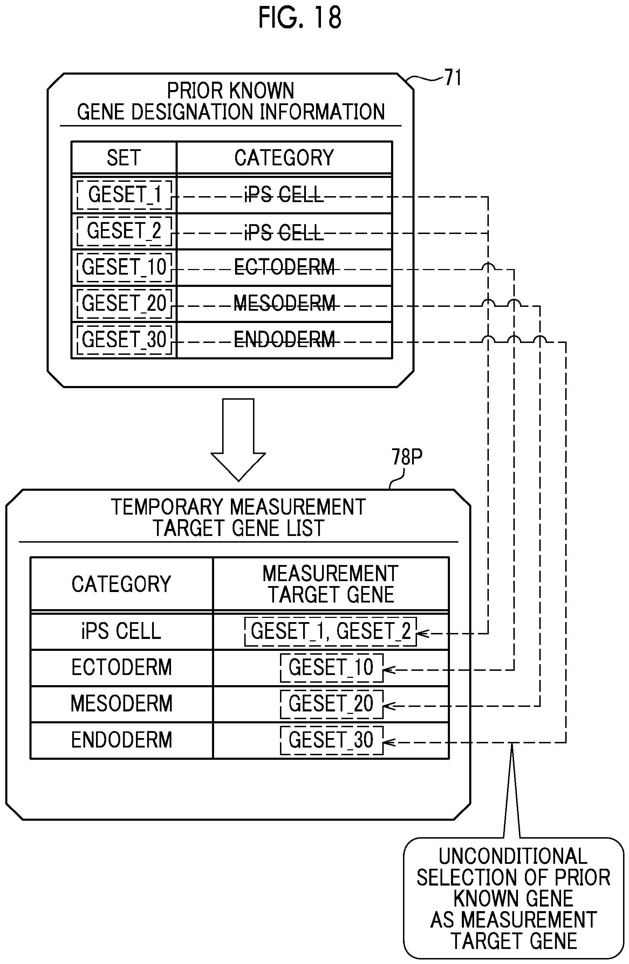

In Fig. 18, first, the selection unit 64 unconditionally selects the set of the prior known genes designated by the prior known gene designation information 71 as the measurement target gene. As a result, a temporary measurement target gene list 78P in which the set of the prior known genes is registered as the measurement target gene is generated. An aspect in which this set of the prior known genes is unconditionally selected as the measurement target gene is an example in which the weighting of the evaluation value of the prior known gene is increased so that the prior known gene is always selected as the measurement target gene.

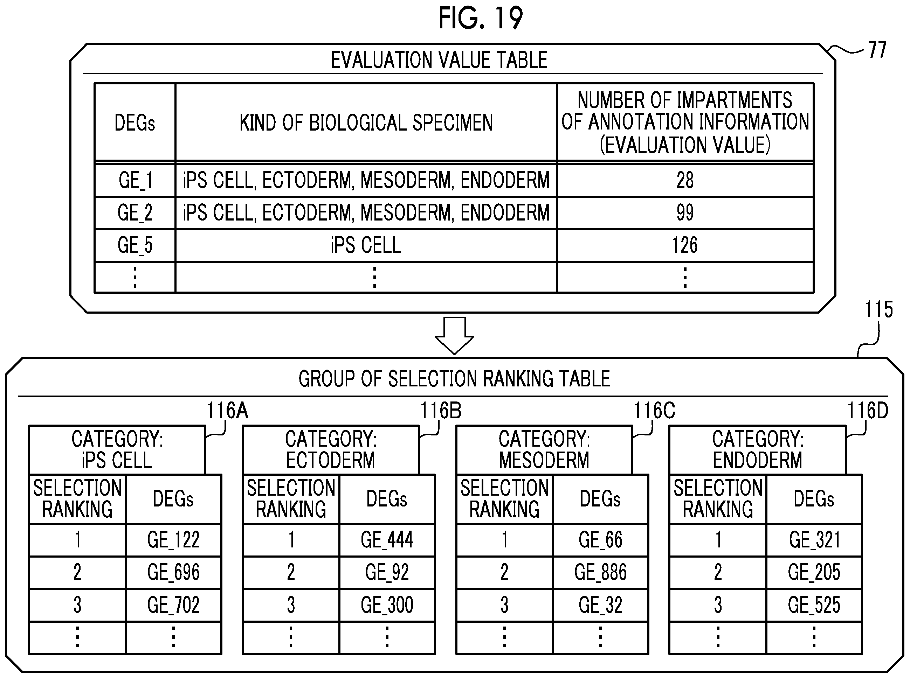

In Fig. 19, the selection unit 64 generates a selection order table group 115 based on the evaluation value table 77. The selection order table group 115 is composed of a selection order table 116A of the category "iPS cell" corresponding to the kind of the biological specimen "iPS cell", a selection order table 116B of the category "ectoderm" corresponding to the kind of the biological specimen "ectoderm", a selection order table 116C of the category "mesoderm" corresponding to the kind of the biological specimen "mesoderm", and a selection order table 116D of the category "endoderm" corresponding to the kind of the biological specimen "endoderm". The selection unit 64 assigns a selection order to each category in order from the DEGs having the highest evaluation value (having the high number of impartments of the annotation information). That is, the selection order of the DEGs having the highest evaluation value is the first place, the selection order of the DEGs having the second highest evaluation value is the second place, the selection order of the DEGs having the third highest evaluation value is the third place, and so on.

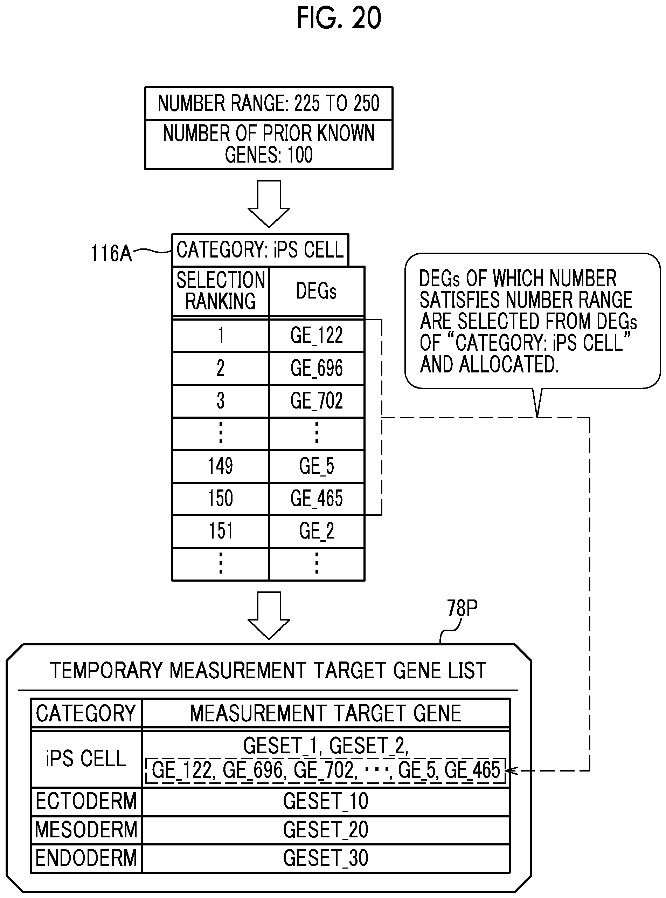

As illustrated in Fig. 20, the selection unit 64 selects measurement target genes satisfying the number range from the DEGs prepared for each category with reference to the selection order table 116 and allocates the genes to each category.

Fig. 20 illustrates a situation in which a measurement target gene of the category "iPS cell" is selected from the DEGs prepared for the category "iPS cell". Fig. 20 illustrates an exemplary case where "225 to 250" illustrated in Fig. 9 is designated as the number range of the category "iPS cell", and the number of prior known genes of the category "iPS cell" selected in Fig. 18 is 100. In this case, in order to satisfy the number range, it is necessary to select at least 125 DEGs and at most 150 DEGs. Therefore, the selection unit 64 selects a total of 150 DEGs from the 1st place to the 150th place in the selection order of the selection order table 116A. Then, the selected 150 DEGs are registered in the temporary measurement target gene list 78P as the measurement target gene of the category "iPS cell".

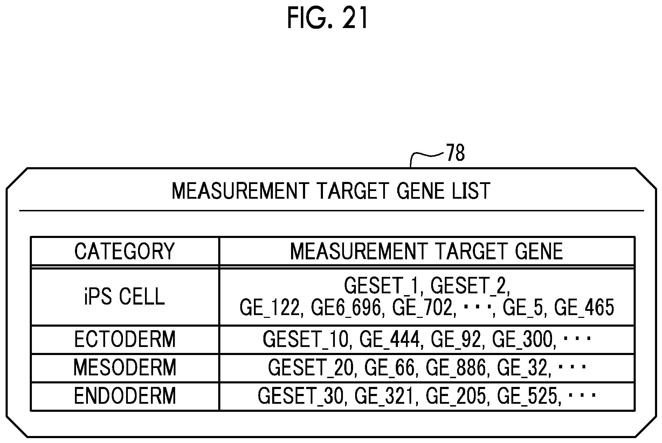

Although not illustrated in the drawing, the selection unit 64 also selects DEGs of which the number satisfies a number range, with reference to the selection order tables 116B to 116D in the same manner for the other categories "ectoderm", "mesoderm", and "endoderm". Then, the selected DEGs are registered in the temporary measurement target gene list 78P as the measurement target gene. By sequentially selecting the measurement target gene in this way, the selection unit 64 finally generates the measurement target gene list 78 which satisfies the number range in each category, as illustrated in Fig. 21.

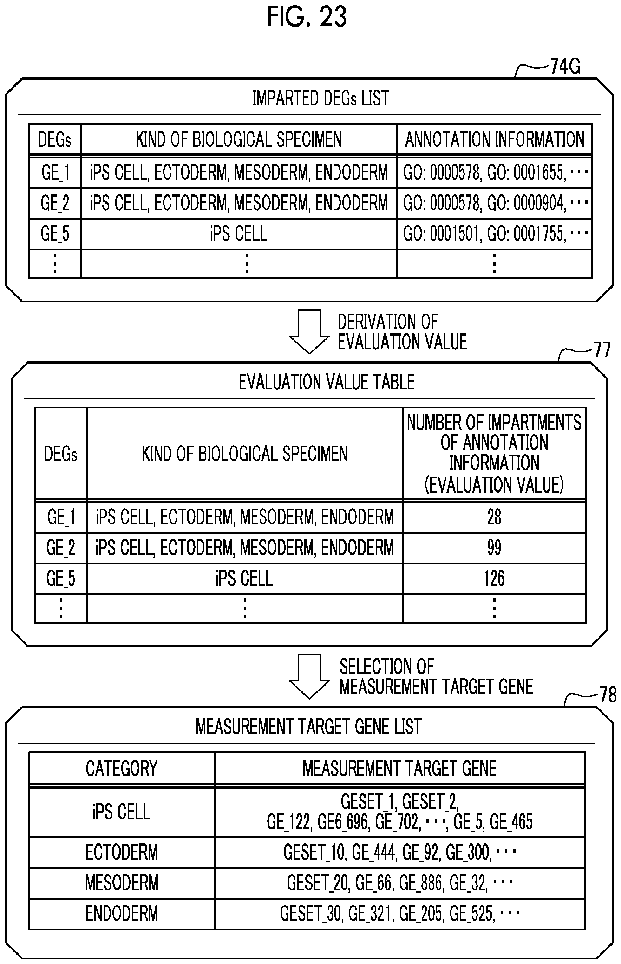

Fig. 22 and Fig. 23 are diagrams summarizing a series of processing by the extraction unit 61, the acquisition unit 62, the derivation unit 63, and the selection unit 64. First, as illustrated in Fig. 22, the extraction unit 61 extracts DEGs from the extraction target 15E and generates the DEGs list 74. The acquisition unit 62 acquires the distribution information 76 from the annotation information DB server 13 to acquire the annotation information. The acquisition unit 62 imparts the annotation information of the distribution information 76 to the DEGs list 74 to obtain the imparted DEGs list 74G.

As illustrated in Fig. 23, the derivation unit 63 counts the number of impartments of the annotation information to each of the DEGs and registers the number of impartments in the evaluation value table 77 as an evaluation value. The selection unit 64 selects the measurement target gene based on the evaluation value and generates the measurement target gene list 78.

As illustrated in Fig. 24, the measurement target gene registered in the measurement target gene list 78 is displayed on the measurement target gene display screen 120. On the measurement target gene display screen 120, each of display regions 121A, 121B, 121C, and 121D is provided for each category. The measurement target gene of the category "iPS cell" is displayed in the display region 121A. The measurement target gene of the category "ectoderm" is displayed in the display region 121B, the measurement target gene of the category "mesoderm" is displayed in the display region 121C, and the measurement target gene of the category "endoderm" is displayed in the display region 121D.

A save button 122, a print button 123, and a confirm button 124 are provided in the lower part of the measurement target gene display screen 120. The save button 122 is selected in a case of saving the measurement target gene list 78. The print button 123 is selected in a case of printing the measurement target gene list 78. In a case of selecting the confirm button 124, the display control unit 65 turns off the display of the measurement target gene display screen 120.

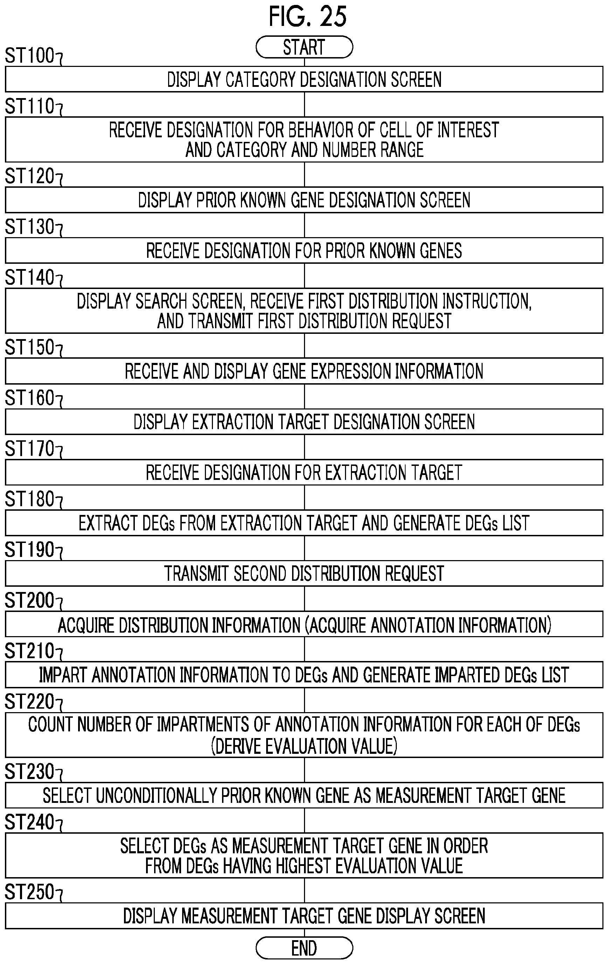

Next, an operation based on the above configuration will be described with reference to the flowchart of Fig. 25. First, in a case where the operation program 55 is started in the information processing apparatus 10, the CPU 47 of the information processing apparatus 10 functions as the instruction receiving unit 60, the extraction unit 61, the acquisition unit 62, the derivation unit 63, the selection unit 64, and the display control unit 65, as illustrated in Fig. 8.

Under the control of the display control unit 65, the category designation screen 80 illustrated in Fig. 9 is displayed on the display 49 (a step ST100). A user inputs the behavior of the cell of interest and a desired category and number range and selects the designation button 86. As a result, the instruction receiving unit 60 receives the designation for the behavior of the cell of interest and the category and the number range (a step ST110), and the category and number range designation information 70 is generated. The category and number range designation information 70 is output from the instruction receiving unit 60 or the selection unit 64.

Subsequently, under the control of the display control unit 65, the prior known gene designation screen 95 illustrated in Fig. 12 is displayed on the display 49 (a step ST120). The user inputs a desired set of prior known genes and selects the designation button 98. As a result, the instruction receiving unit 60 receives the designation for the set of the prior known genes (a step ST130), and the prior known gene designation information 71 is generated. The prior known gene designation information 71 is output from the instruction receiving unit 60 to the selection unit 64.

Under the control of the display control unit 65, a search screen, which is not illustrated in the drawing, is displayed on the display 49. Then, the instruction receiving unit 60 receives the first distribution instruction by the user, which includes the search keyword. As a result, the instruction receiving unit 60 transmits the first distribution request 72 including the search keyword to the gene expression information DB server 12 (a step ST140).

The gene expression information 15 is distributed from the gene expression information DB server 12 in response to the first distribution request 72. The gene expression information 15 is input to the display control unit 65. Then, under the control of the display control unit 65, a display screen of the gene expression information 15, which is not illustrated in the drawing, is displayed on the display 49 (a step ST150).

In addition, under the control of the display control unit 65, the extraction target designation screen 105 illustrated in Fig. 13 is displayed on the display 49 (a step ST160). The user inputs the desired extraction target 15E and selects the designation button 108. As a result, the instruction receiving unit 60 receives the designation for the extraction target 15E (a step ST170) and generates the extraction target designation information 73. The extraction target designation information 73 is output from the instruction receiving unit 60 to the extraction unit 61.

In the extraction unit 61, DEGs are extracted from the extraction target 15E, and the DEGs list 74 illustrated in Fig. 14 is generated (a step ST180). The DEGs list 74 is output from the extraction unit 61 to the acquisition unit 62. Subsequently, the second distribution request 75 based on the DEGs list 74 is transmitted from the acquisition unit 62 to the annotation information DB server 13 (a step ST190).

In response to the second distribution request 75, the distribution information 76 including the annotation information illustrated in Fig. 15 is distributed from the annotation information DB server 13. The distribution information 76 is input to the acquisition unit 62. As a result, the distribution information 76 and, by extension, the annotation information are acquired by the acquisition unit 62 (a step ST200). The step ST200 is an example of "acquisition processing".

As illustrated in Fig. 16, the annotation information is imparted to the DEGs list 74 based on the distribution information 76 by the acquisition unit 62, and the DEGs list 74 is set to the imparted DEGs list 74G (a step ST210). At this time, only the annotation information related to the behavior of the cell of interest is selected and imparted. The imparted DEGs list 74G is output from the acquisition unit 62 to the derivation unit 63.

As illustrated in Fig. 17, the derivation unit 63 counts the number of impartments of the annotation information imparted to each of the DEGs, and the number of impartments is registered in the evaluation value table 77 as an evaluation value (a step ST220). The evaluation value table 77 is output from the derivation unit 63 to the selection unit 64. The step ST220 is an example of "derivation processing".

As illustrated in Fig. 18, the selection unit 64 unconditionally selects the prior known gene as the measurement target gene (a step ST230).

Further, as illustrated in Fig. 20, the selection unit 64 selects DEGs of which the number satisfies a number range, from the DEGs prepared for each category in descending order of the evaluation values. Then, the selected DEGs are allocated to each category as the measurement target genes (a step ST240). Through such a process, the measurement target gene list 78 illustrated in Fig. 21 is generated. The measurement target gene list 78 is output from the selection unit 64 to the display control unit 65. The step ST240 is an example of "selection processing".

Finally, the display control unit 65 displays the measurement target gene display screen 120, illustrated in Fig. 24, on the display 49 (a step ST250). The user confirms the measurement target gene through the measurement target gene display screen 120.

As described above, the information processing apparatus 10 includes the acquisition unit 62, the derivation unit 63, and the selection unit 64. The acquisition unit 62 acquires the annotation information imparted to each of the plurality of genes. The derivation unit 63 derives an evaluation value for each of the plurality of genes based on the annotation information. The selection unit 64 selects a measurement target gene from among the plurality of genes based on the evaluation value. As a result, it is possible to select the measurement target gene in a data-driven manner under the reliable support of the evaluation value based on the annotation information. The measurement target gene selected in this manner is customized according to the cell as a research target while being easily developed in multilevel. This makes it possible to select a more proper measurement target gene, which leads to the elucidation of the behavior of the cell.

The acquisition unit 62 selects annotation information related to the behavior of the cell of interest. The selection unit 64 derives an evaluation value based only on the selected annotation information. This makes it possible to select the measurement target gene based only on the annotation information specific to the behavior of the cell of interest. In other words, it is possible to exclude annotation information having a low relevance to the behavior of the cell of interest as noise and select a measurement target gene in a form limited to the annotation information having a high relevance to the behavior of the cell of interest.

The acquisition unit 62 imparts the annotation information to the gene with reference to the annotation information DB 16 in which the annotation information for the gene is registered. This makes it possible to easily impart the annotation information by using the existing annotation information DB 16.

The annotation information is associated with the kind of the biological specimen (for example, the kind of the cell). The instruction receiving unit 60 receives, the designation by a user for a plurality of categories defined according to the kind of the biological specimen and for a number range for each of the plurality of categories. The selection unit 64 selects genes of which the number satisfies a number range, from the genes prepared for each of the plurality of categories, and it allocates the selected genes to each of the plurality of categories as the measurement target gene. This makes it possible to select the measurement target gene without excess or deficiency for each category.

In a case where the expression level of the gene is measured for the intended purpose of evaluating the iPS cell 25 and a differentiation process thereof, the category includes, for example, "iPS cell", "ectoderm", "mesoderm", and "endoderm". In this case, it is possible to obtain the measurement target gene for each category associated with the iPS cell 25. The category is not limited to those described above, and it is preferably made to be proper depending on the intended purpose. In a case where the expression level of the gene is measured for the intended purpose of evaluating the ES cell and a differentiation process thereof, the category includes, for example, "ES cell", "ectoderm", "mesoderm", and "endoderm".

The derivation unit 63 counts the number of impartments of the annotation information for each of the plurality of genes and derives an evaluation value based on the number of impartments. This makes it possible to easily derive the evaluation value.

The gene includes a prior known gene. Then, the instruction receiving unit 60 receives the designation by a user for the prior known gene. The selection unit 64 unconditionally selects the prior known gene as the measurement target gene as one form in which the weighting of the evaluation value of the prior known gene is increased. This makes it possible to reflect a user's intention of desiring to measure the prior known gene. In addition, it is possible to effectively incorporate the prior known gene in which the past findings are condensed.

The selection unit 64 selects more than 100 and 1,000 or fewer measurement target genes. A case where the number of the measurement target genes is more than 100 is advantageous for elucidating the behavior of the cell. On the other hand, in a case where the number of the measurement target genes is 1,000 or less, the time and cost of the test are not too excessive, which is advantageous for the development into a multilevel experiment.

The gene includes DEGs. This makes it possible to select a measurement target gene that is conceived to contribute to the further elucidation of the behavior of the cell.

As described above, the prior known gene is unconditionally selected as the measurement target gene; however, the present invention is not limited to this. Similarly to the case of the DEGs, annotation information may be acquired to derive an evaluation value for the prior known gene, and the prior known gene may be also selected based on the derived evaluation value. At this time, the weighting of the evaluation value of the prior known gene may be increased as compared with the case of the DEGs. In addition, in this case, the importance may be set for each prior known gene, and the evaluation value may be derived in consideration of the importance. Specifically, the higher the importance is, the higher the evaluation value is configured to be derived. Regarding genes other than the prior known genes, for example, DEGs, the evaluation value may be derived by assuming that the importance is the lowest.

The prior known gene does not necessarily have to be designated. For example, in a case where the cell as a research target is novel and the prior known gene is not present in the first place, the designation for the prior known gene may be omitted.

The designation for the extraction target 15E may also be omitted, and the entire gene expression information 15 distributed from the gene expression information DB server 12 may be set as the extraction target 15E.

The category does not necessarily have to be designated either. However, even in a case where the designation for the category is omitted, it is necessary to designate a range, at least an upper limit, of the number of measurement target genes to be selected.

The gene expression information DB 14 is not limited to a public DB such as the exemplary GEO. For example, it may be a local DB in which the gene expression information 15 measured at the laboratory to which a user belongs is registered. Similarly, the annotation information DB 16 is not limited to a public DB such as DAVID or InterPro, and it may be, for example, a local DB prepared at a laboratory to which a user belongs.

### [Second embodiment]

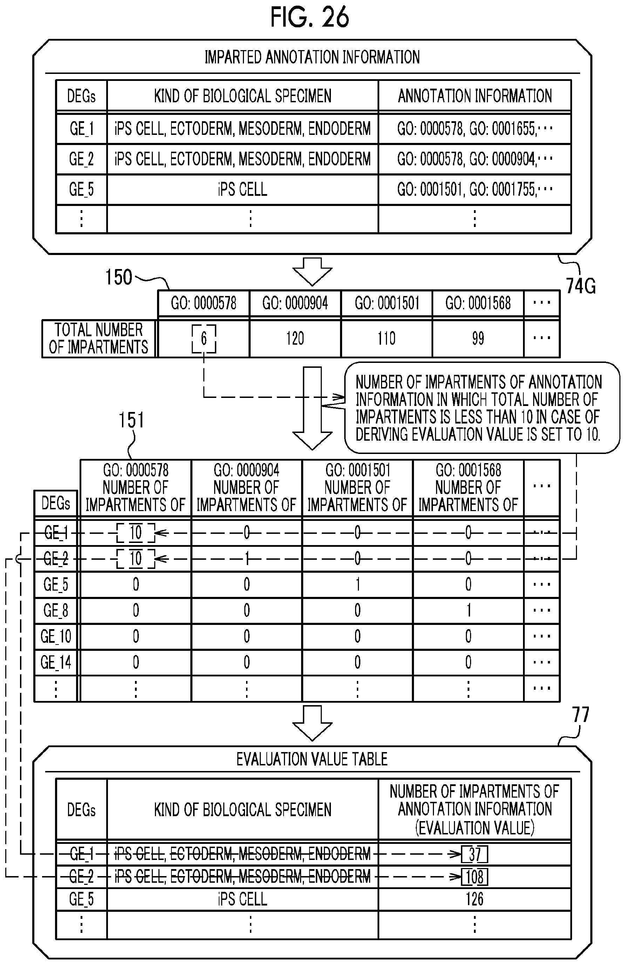

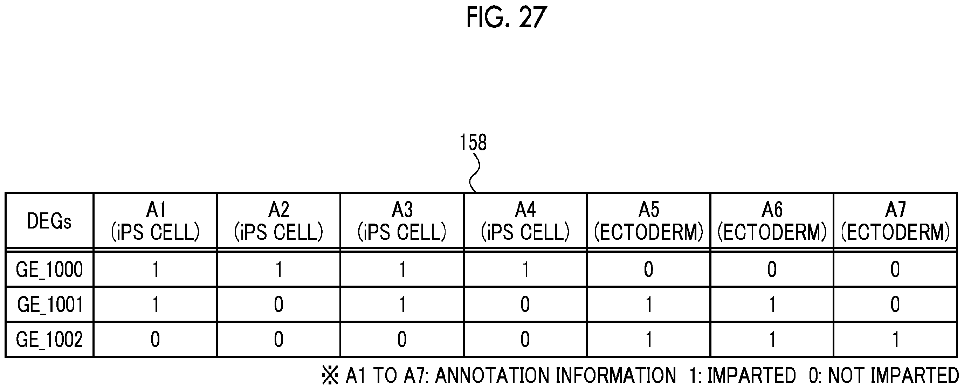

In the second embodiment illustrated in Fig. 26 and Fig. 27, the weighting of the evaluation value is carried out according to the information value of the annotation information.

Fig. 26 is a diagram showing an example in which annotation information having a relatively small number of impartments and having a relatively high rarity is determined to have a high information value and the number of impartments of the annotation information is increased. First, as shown in Table 150, the derivation unit 63 counts the number of impartments of each annotation information imparted to the DEGs (hereinafter, referred to as the total number of impartments) based on the imparted DEGs list 74G. The derivation unit 63 compares the total number of impartments with a preset threshold value. Then, it is determined that the annotation information in which the total number of impartments is smaller than the threshold value has a high information value, and as shown in Table 151, the number of impartments of the annotation information in a case of deriving the evaluation value is set to a value larger than 1. That is, the weighting of the annotation information determined to have a high information value is increased. The derivation unit 63 counts the number of impartments of the annotation information of each of the DEGs, including the number of impartments after the weighting, and generates the evaluation value table 77.

Fig. 26 illustrates an exemplary case where "10" is set as the threshold value and the number of impartments of the annotation information of ID "GO: 0000578" having the total number of impartments of "6" and less than the threshold value is set to "10".

Fig. 27 shows an example in which the weighting of the evaluation value is carried out based on the orthogonality of the annotation information. The derivation unit 63 finds a set of genes, which can cover the annotation information without omission and duplication as little as possible, and it determines that the orthogonality of the annotation information is high with respect to the set of the genes.

The table 158 illustrates an exemplary table in which the impartment circumstance of the annotation information indicated by A1 to A7 is shown with respect to the three DEGs of IDs "GE_1000", "GE_1001", and "GE_1002". Among the annotation information of A1 to A7, "iPS cell" is associated in each of A1 to A4 as the kind of biological specimen, and "ectoderm" is associated in each of A5 to A7.

In this case, in a case of looking at only the number of impartments of the annotation information, the DEGs of ID "GE_1000" and ID "GE_1001" are preferentially selected as the measurement target genes as compared with the DEGs of ID "GE_1002". However, in consideration of the orthogonality of the annotation information, the DEGs of ID "GE_1002" are preferentially selected as the measurement target genes as compared with the DEGs of ID "GE_1001". In this way, in a case where the DEGs of IDs "GE_1000" and ID "GE_1002" are finally selected as the measurement target genes, both the "iPS cell" and the "ectoderm" can be covered.

The evaluation value may be derived based on the number of annotation information that can be covered by a combination with other genes. Giving a description using Table 158 as an example, the number of annotation information that can be covered is 6 in the combination of the DEGs of ID "GE_1000" and ID "GE_1001". In the combination of the DEGs of ID "GE_1000" and ID "GE_1002", the number of annotation information that can be covered is 7. In the combination of the DEGs of ID "GE_1001" and ID "GE_1002", the number of annotation information that can be covered is 5. From this result, the evaluation value of the DEGs of ID "GE_1000" and ID "GE_1002" is set to be higher than the evaluation value of the DEGs of ID "GE_1001".

As described above, in the second embodiment, the derivation unit 63 carries out the weighting of the evaluation value according to the information value of the annotation information. For this reason, for example, in a case of increasing the weighting of the number of impartments of the annotation information determined to have a high information value, it becomes easy to select a gene to which the annotation information having a high information value is imparted, as the measurement target gene. This makes it possible to increase the validity and reliability of the measurement target gene.

In Fig. 26, the derivation unit 63 determines that the annotation information having a relatively high rarity has a high information value and increases the weighting of the annotation information. This makes it possible to select a gene to which rare annotation information which tends to be overlooked is imparted, as the measurement target gene.

In Fig. 27, the derivation unit 63 carries out the weighting of the evaluation value based on the orthogonality of the annotation information. This makes it possible to select a set of genes, which can cover the annotation information without omission and duplication as little as possible, as the measurement target gene.

The examples of Fig. 26 and Fig. 27 may be combined and carried out. In this case, for example, the annotation information in which the total number of impartments is smaller than the threshold value is imparted, and 100 is added to the evaluation value of the DEGs in which the orthogonality of the annotation information is high.

In Fig. 26, the annotation information having a relatively high rarity is determined to be the annotation information having a high information value; however, examples of the annotation information having a high information value are not limited thereto. For example, annotation information having a relatively large number of publications in research papers may be determined to be annotation information having a high information value.

In Fig. 26, the number of impartments of the annotation information imparted to the DEGs is subjected to weighting; however, the aspect of weighting is not limited to this. In a case where the evaluation value is also derived for the prior known gene, the number of impartments of the annotation information imparted to the prior known gene may be subjected to weighting in the same manner as in the case illustrated in Fig. 26. Similarly, the aspect illustrated in Fig. 27 may be also applied to the prior known gene.

### [Third embodiment]

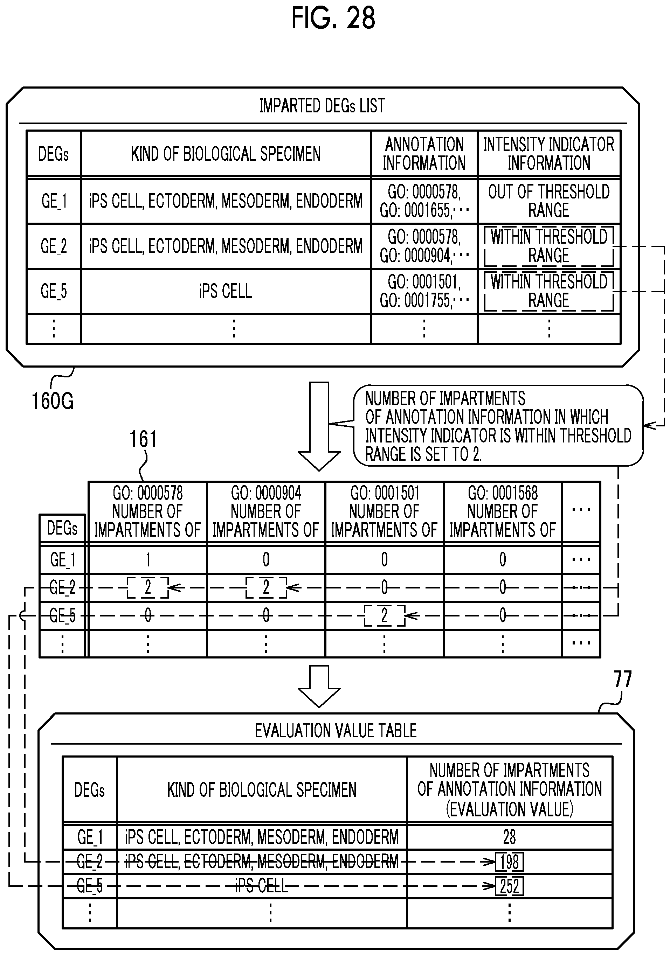

In the third embodiment illustrated in Fig. 28, the weighting of the evaluation value of the gene of which the intensity indicator is within a preset threshold range is increased.

In Fig. 28, the item of the intensity indicator information is provided in the imparted DEGs list 160G according to the third embodiment. In the item of the intensity indicator information, whether or not the intensity indicator is within a preset threshold range is registered. The intensity indicator is, for example, a fold-change, a q value (q-value) that indicates a significant difference in expression corrected by multiple tests.

As shown in Table 161, the derivation unit 63 sets the number of impartments in a case of deriving the evaluation value to a value larger than 1, where the number of impartments is that of the annotation information of the DEGs of which the intensity indicator is within a threshold range. That is, the weighting of the evaluation value of the DEGs of which the intensity indicator is within a threshold range is increased. The derivation unit 63 counts the number of impartments of the annotation information of each of the DEGs, including the number of impartments after the weighting, and generates the evaluation value table 77.

Fig. 28 illustrates an exemplary case where the intensity indicator of DEGs such as IDs "GE_2" and "GE_5" is within a threshold range and the number of impartments of the annotation information is "2".

As described above, in the third embodiment, the derivation unit 63 increases the weighting of the evaluation value of the DEGs of which the intensity indicator is within a threshold range. This makes it possible to select DEGs of which the intensity indicator is within a threshold range, as the measurement target gene, where the DEGs are conceived to be important for elucidating the characteristics of the biological specimen (for example, the characteristics of the cell). The second embodiment and the third embodiment may be combined and carried out.

As described above, the measurement target biomarker can be selected with the support of the evaluation value based on the annotation information. The measurement target biomarker selected in this way is customized according to the kind of the cell of interest. This makes it possible to select a more proper measurement target biomarker, which is linked to the culture step of the cell A or the adjustment of the phenotype of the cell B.

### [Step (2)]

In the step (2), the evaluation data of the measurement target biomarker is acquired from at least one of the cell A or the culture system, at least before the start of culture of the cell A or during the culture. In the step (2), a specimen may be sampled from at least one of the cell A or the culture system, at least before the start of culture of the cell A or during the culture, the sampled specimen may be stored, and the evaluation data of the measurement target biomarker may be acquired from the stored specimen. The biomarker to be a measurement target in the step (2) is the biomarker selected in the step (1).

The step (2) is carried out at least before the start of the culture of the cell A or during the culture. Before the start of the culture of the cell A, the measurement specimen is, for example, the cell A itself, an extract from the cell A, a secretion of the cell A, or the like. During the culture of the cell A, the measurement specimen is, for example, the cell A itself, an extract from the cell A, a culture solution, a gas in the culture system, or the like.

The step (2) may be carried out once or a plurality of times. In the step (2), a specimen may be sampled from at least one of the cell A or the culture system, at a plurality of time points, the sampled specimen may be stored, and the evaluation data of the measurement target biomarker may be acquired from the stored specimen.

In a case where the step (2) is carried out at a plurality of time points, A biomarker for which evaluation data is acquired at a certain time point and a biomarker for which evaluation data is acquired at another time point may be the same or different from each other. It is preferable to acquire evaluation data for all the measurement target biomarkers through a plurality of time points.

The culture of the cell A is carried out under culture conditions suitable for the survival of the cell A. In addition, during the culture of the cell A, the cell A is induced to differentiate into the cell B as necessary, and a culture suitable for inducing the differentiation of the cell B, that is, a differentiation culture is carried out. The expansion culture of the cell A may be carried out before the culture for the differentiation of the cell A into the cell B.

In a case where the step (2) is carried out during the culture of the cell A, it may be carried out during the expansion culture of the cell A, that is, before the start of the differentiation culture, or it may be carried out during the culture for the differentiation of the cell A into the cell B. The step (2) can be carried out, for example, in a period from 3 days before the start of the differentiation culture to 14 days after the start of the differentiation culture. It is preferably carried out in a period from 3 days before the start of the differentiation culture to 10 days after the start of the differentiation culture and more preferably carried out in a period from the day before the start of the differentiation culture to 8 days after the start of the differentiation culture. In addition, the step (2) can be carried out, for example, in a period from 3 days before the start of the differentiation culture to the day before the end of the differentiation culture. It is preferably carried out in a period from 3 days before the start of the differentiation culture to 3 days before the end of the differentiation culture and more preferably carried out in a period from the day before the start of the differentiation culture to 5 days before the end of the differentiation culture.

### [Step (3)]

In the step (3), the evaluation data of the discrimination marker of the B cell is acquired from at least one of the cell A or the culture system, at least at the final stage of the culture of the cell A or after the end of the culture. In the step (3), a specimen may be sampled from at least one of the cell A or the culture system, at least at the final stage of the culture of the cell A or after the end of the culture, the sampled specimen may be stored, and the evaluation data of the discrimination marker of the cell B may be acquired from the stored specimen. The "final stage" and "after the end of the culture" mean a time at which the proliferation, morphological change, or cell differentiation of the cell A has progressed to the extent that the discrimination marker of the cell B can be detected.

The kind of the discrimination marker of the cell B is not limited as long as it is a marker derived from the cell B and thus the presence or absence of the cell B can be confirmed. The discrimination marker of the cell B may be one kind or two or more kinds. Examples of the discrimination marker of the cell B include the presence or absence of expression of a gene, the expression level of a gene, the presence or absence of expression of a protein, the expression level of a protein, and the morphology of a cell. From the viewpoint that an immunological detection method can be applied, the discrimination marker of the cell B is preferably the presence or absence or the amount of a protein specifically expressed in the cell B.

The step (2) and the step (3) may be carried out on one clone of the cell A or may be carried out on a plurality of clones of the cell A.

The culture of the cell A may be carried out once or a plurality of times, whereby the step (2) and the step (3) may be carried out once or may be carried out a plurality of times.

In a case of the culture of a plurality of clones or a plurality of times of the culture, a plurality of sets of the evaluation data set of the evaluation data of the measurement target biomarker and the evaluation data of the discrimination marker of the cell B are acquired. A plurality of sets of the evaluation data set may be subjected to the step (4).

### [Step (4)]

In the step (4), at least one biomarker indicating characteristics of the cell A that is used for producing the cell B is identified from among the measurement target biomarkers, based on the evaluation data of the measurement target biomarker and the evaluation data of the discrimination marker of the cell B. In the step (4), at least one biomarker indicating the characteristics of the cell A and having a relationship with the cell A and the cell B is identified from among the measurement target biomarkers.

### Examples of the embodiment of the step (4) include the following one.

A plurality of sets of the evaluation data set of the evaluation data of the measurement target biomarker and the evaluation data of the discrimination marker of the cell B are acquired. Based on the evaluation data of the discrimination marker of the cell B, at least each of one cell A having a high production efficiency of the cell B and one cell A having a low production efficiency of the cell B is selected. The evaluation data of the measurement target biomarker is compared between the cell A having a high production efficiency of the cell B and the cell A having a low production efficiency of the cell B, and at least one biomarker having a significant difference in the expression level, the generation amount, the concentration, and the like is identified.

In a case where the number of measurement target biomarkers is relatively large, it is possible, in the step (4), to analyze the evaluation data of the measurement target biomarker and the evaluation data of the discrimination marker of the cell B with, for example, a statistical method or machine learning. Any statistical method or machine learning can be applied for carrying out the step (4). Examples of the statistical method or machine learning include a regression analysis using a random forest, a logistic regression analysis, neural networking, a support vector machine, naive bays, and a decision tree. It is preferable that the step (4) is carried out by being processed by a computer.

An example of the embodiment of the step (4) is the regression analysis using a random forest. The random forest is one of the machine learning algorithms. Examples of the embodiment of the step (4) to which the regression analysis using the random forest is applied include the following one.

A plurality of sets of the evaluation data set of the evaluation data of the measurement target biomarker and the evaluation data of the discrimination marker of the cell B are acquired. The evaluation data of the measurement target biomarker are set as the explanatory variables, and the evaluation data of the discrimination marker of the cell B are set as the response variables. Two or more sets of the evaluation data set are used as training data to create a prediction model of the discrimination marker of the cell B. Another evaluation data set that has not been subjected to the creation of the prediction model is used as test data to check the accuracy of the prediction model. In the prediction model confirmed to have high accuracy, an item having a high degree of contribution to the creation of the prediction model is extracted from among the explanatory variables, and the extracted item is used as the biomarker indicating the characteristics of the cell A.

In a case where the explanatory variables are excessively present with respect to the response variables in carrying out the step (4), the data amount of the explanatory variables may be compressed and/or reduced by using a known dimension reduction means. As the means for compressing and/or reducing the data amount, it is possible to use a sparse modeling method such as a principal component analysis (PCA), a latent semantic analysis (LSA), a linear discriminant analysis (LDA), or Lasso.

In a case where the explanatory variable has a missing value in carrying out the step (4), the missing value may be substituted by using a known missing value complementation method. As the missing value complementation method, it is possible to use gradient boosting, linear complementation, or the like.

### <Cell producing method>

The cell producing method according to the present disclosure is a method of culturing the cell A to produce the cell B, where the cell producing method includes the following (A) to (C): The following (A) and the like are also referred to as a step (A) and the like, respectively.
(A) Carrying out the biomarker identifying method according to the present disclosure to identify at least one biomarker indicating characteristics of the cell A.
(B) Identifying at least one of a signal transduction in which the biomarker is involved or a mechanism by which the biomarker varies with reference to annotation information of the identified biomarker.
(C) Culturing the cell A under culture conditions, in which at least one of the signal transduction or the mechanism is inhibited or promoted, to produce the cell B.

In the cell producing method according to the present disclosure, a biomarker indicating the characteristics of the cell A that is used for producing the cell B can be rapidly identified by carrying out the steps (A) to (C), and the production step can be rapidly optimized based on the annotation information of the biomarker.

The kind of the cell A and the kind of the cell B are as described above. In an example of the embodiment of the present disclosure, the cell A is a pluripotent stem cell such as an ES cell or an iPS cell, and the cell B is a differentiated cell derived from the pluripotent stem cell.

### [Step (A)]

In the step (A), the biomarker identifying method according to the present disclosure is carried out to identify at least one biomarker indicating characteristics of the cell A. The details of the step (A) are as described in the above-described steps (1) to (4).

### [Step (B)]

In the step (B), at least one of the signal transduction in which the biomarker is involved or the mechanism by which the biomarker varies is identified with reference to annotation information of the identified biomarker.

The identified biomarker has annotation information. In a case where a plurality of identified biomarkers are present or a plurality of pieces of annotation information are imparted to one biomarker, so that a plurality of pieces of annotation information to be referred to are present, it is preferable that the annotation information having a relatively high information value is referenced. The annotation information having a relatively high information value is, for example, the annotation information having a relatively high rarity, the annotation information having a relatively high orthogonality to the cell B, the annotation information having a relatively large number of publications in research papers, and the like.

In order to identify at least one of the signal transduction in which the biomarker is involved or the mechanism by which the biomarker varies, the following analysis may be carried out; for example, carrying out a query of the biomarker to a public database, finding commonality between a plurality of pieces of annotation information, identifying a signal transduction pathway from the annotation information by an enrichment analysis.

### [Step (C)]

In the step (C), the cell A is cultured under culture conditions, in which at least one of the signal transduction or the mechanism is inhibited or promoted, to produce the cell B. The step (C) is a step of inhibiting or promoting at least one of the signal transduction or the mechanism, which is identified in the step (B), and linking it to the culture step of the cell A or the adjustment of the phenotype of the cell B.

The step (C) may be, for example, the following step. The following is an example and thus does not limit the embodiment of the present disclosure.
· The cell A is cultured in a culture medium, to which a protein involved in at least one of the upstream or the downstream of the signal transduction is added, to produce the cell B.
· The cell A is cultured in a culture medium, to which a chemical substance inhibiting or promoting a protein involved in at least one of the upstream or the downstream of the signal transduction is added, to produce the cell B.
· The cell A is cultured in a culture medium, to which a chemical substance inhibiting or promoting a mechanism by which the biomarker varies is added, to produce the cell B.
· The cell A is cultured in a culture medium, in which the biomarker is varied, to produce the cell B.
· The cell A is cultured by adjusting culture conditions under which the biomarker is caused to vary, the culture conditions being, for example, the O₂ concentration in the culture system and the rotation speed of the stirrer for stirring the culture medium, to produce the cells B.

The step (C) may overlap with the step (A) or the step (B) in terms of time, or it may not overlap with the step (A) and the step (B) in terms of time. For example, the step (C) may be started during the step (A) or the step (B), and the culture conditions may be changed at any time point in the step (C).

The step (C) is carried out under culture conditions suitable for the survival of the cell A. Then, during the step (C), the cell A is induced to differentiate into the cell B as necessary, and the culture suitable for inducing the differentiation of the cell B, that is, differentiation culture is carried out. In the step (C), the expansion culture of the cell A may be carried out before the culture for the differentiation of the cell A into the cell B.

### Examples

Hereinafter, the embodiment of the present disclosure will be more specifically described according to Examples; however, the embodiment of the present disclosure is not limited to the following Examples.

The abbreviations for chemical substances and the like are as follows.
bFGF: basic Fibroblast Growth Factor
BMP4: Bone Morphogenetic Protein 4
cTnT: cardiac Troponin T
DMEM: Dulbecco's Modified Eagle Medium
D-PBS: Dulbecco's Phosphate-Buffered Saline
EDTA: Ethylenediaminetetraacetic acid

In the following description, regarding the substance concentration, "%" is "v/v%" unless otherwise specified.

### <Selection of biomarker to be measurement target>

Examples in which a biomarker to be measured is selected will be described, where the cell of interest is set to "iPS cell" and the behavior of the cell of interest is set to "differentiation potency". In the present Example, the category and the number range are respectively the same as those illustrated in Fig. 9. That is, "iPS cell", "ectoderm", "mesoderm", and "endoderm" were designated as the category, and "225 to 250" was designated for each category as the number range.

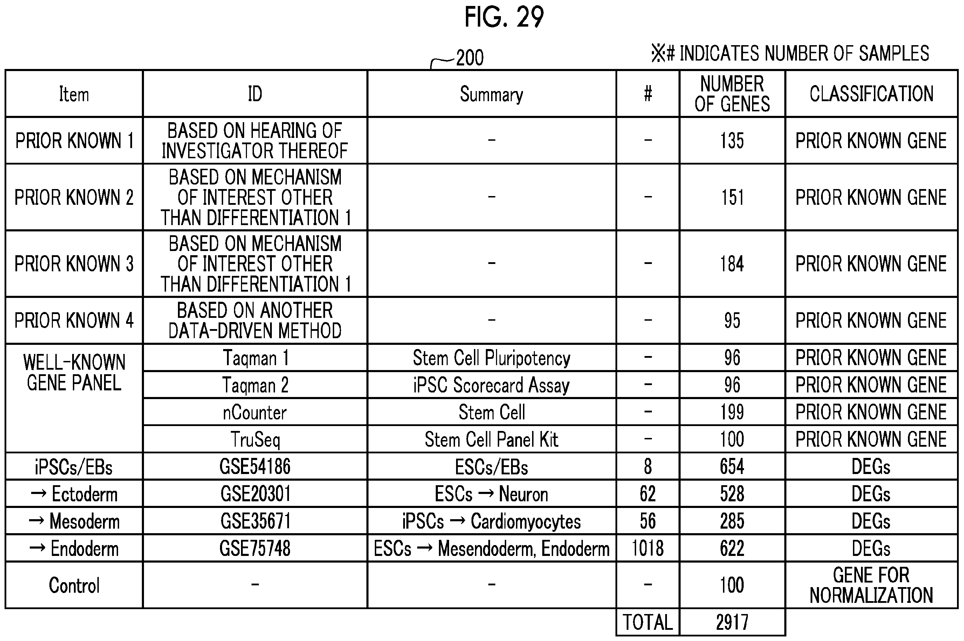

Table 200 shown in Fig. 29 shows the prior known genes designated for selecting measurement target genes in the present Example and extracted DEGs. The prior known gene also includes prior known genes based on the hearing of investigators thereof or a well-known gene panel such as the TaqMan scorecard. The DEGs include the iPS cell 25 or an ES cell, and those extracted from the extraction target 15E in an experiment in which the iPS cell 25 or the ES cell was allowed to differentiate into three germ layers 26 or the tissue cells 30. In the present Example, from among these about 2,900 genes (partially duplicated), about 1,000 (specifically, 980) measurement target genes, which satisfy the number range, were selected. More specifically, only the annotation information related to differentiation among the annotation information acquired from the annotation information DB 16 was selected and imparted to the prior known gene and the DEGs. Then, the evaluation values were derived based on the annotation information, and those of which the number satisfies the number range were selected in descending order of the evaluation values. In addition, a gene for normalization was also selected as a measurement target gene, in addition to the prior known genes and DEGs. In this way, about 1,000 genes (specifically, 980 genes) were selected as the measurement targets. About 1,000 measurement target genes selected are referred to as C1000.

In an experiment of inducing differentiation of the iPS cell 25 into the myocardial cell, the expression level of C1000 in 15 samples at the stage of the iPS cell 25 was measured. Among the 15 samples, 10 samples had a high differentiation induction efficiency, while 5 samples had a low differentiation induction efficiency.

Here, in order to check the effect of the technology of the present disclosure, as Comparative Example, 15 samples of the iPS cell 25 before differentiation induction were separately subjected to the measurement of the comprehensive gene expression level from a microarray (the measurement of the expression level of about 21,000 genes).

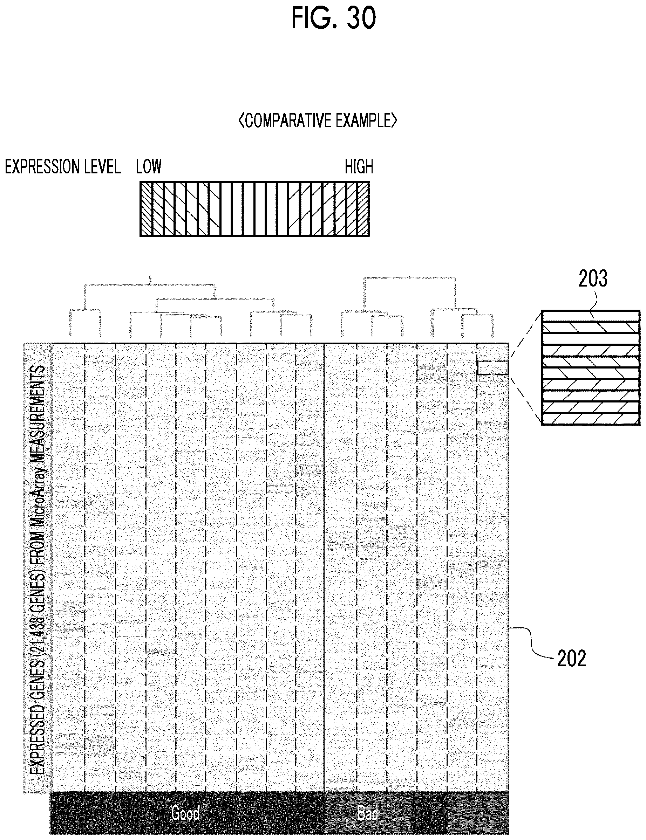

Fig. 30 shows measurement results of gene expression level from microarray 202. A bar 203 represents the expression level of each gene. According to the measurement result 202, the samples were divided by clustering into a group of 9 samples on the left side and a group of 6 samples on the right side, and all 5 samples having a low differentiation induction efficiency, indicated by "Bad", were included in the group of 6 samples. That is, according to the measurement results of gene expression level from microarray 202, it was found that it is possible to predict the highness or lowness of differentiation induction efficiency at the stage of the iPS cell 25 with relatively high accuracy (the detection sensitivity of the sample in which the differentiation induction efficiency is low is 100%, and the specificity of the sample in which the differentiation induction efficiency is low is 83%). "Good" indicates a sample in which the differentiation induction efficiency is high.

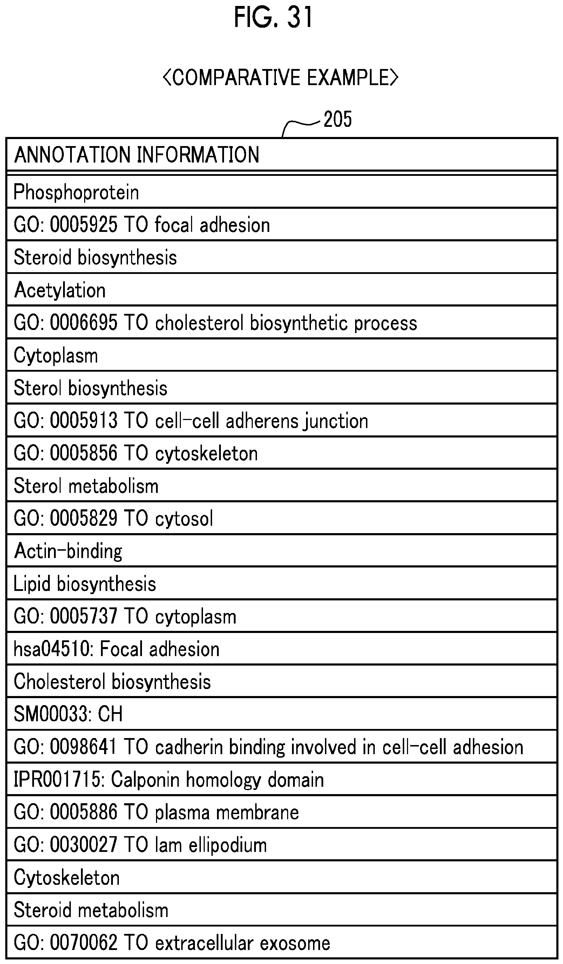

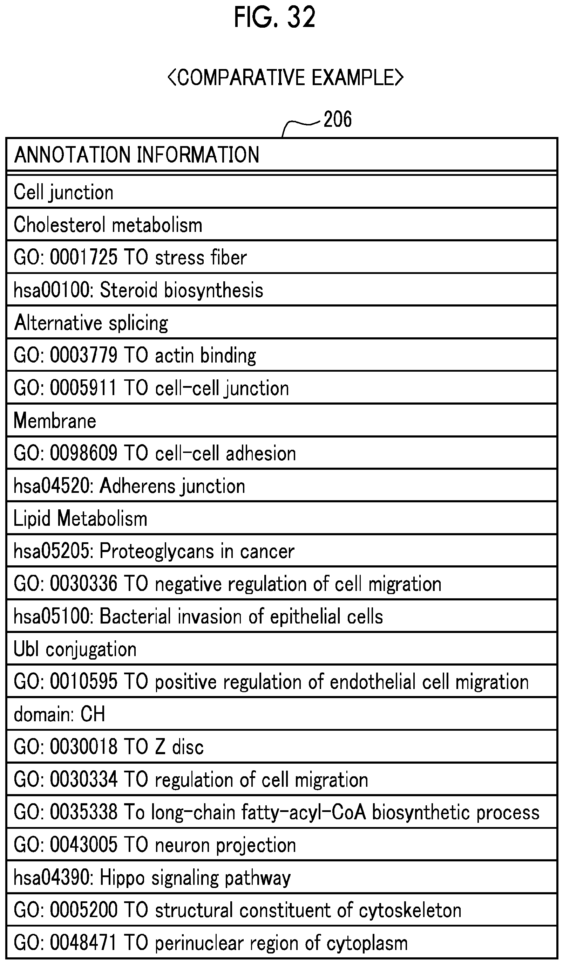

Highly expressed genes having an expression level equal to or higher than the threshold value were extracted from about 21,000 genes that had been measurement targets for the microarray, and the annotation information (referred to as the high-influence annotation information) having a relatively high degree of influence on the behavior of the cell was selected by a statistical method from among the annotation information imparted to the highly expressed genes. The results are shown in Table 205 of Fig. 31 and Table 206 of Fig. 32. According to Tables 205 and 206, it was found that various miscellaneous annotation information was selected as the high-influence annotation information, and it is difficult to obtain effective findings that lead to the elucidation of the behavior of the cell.

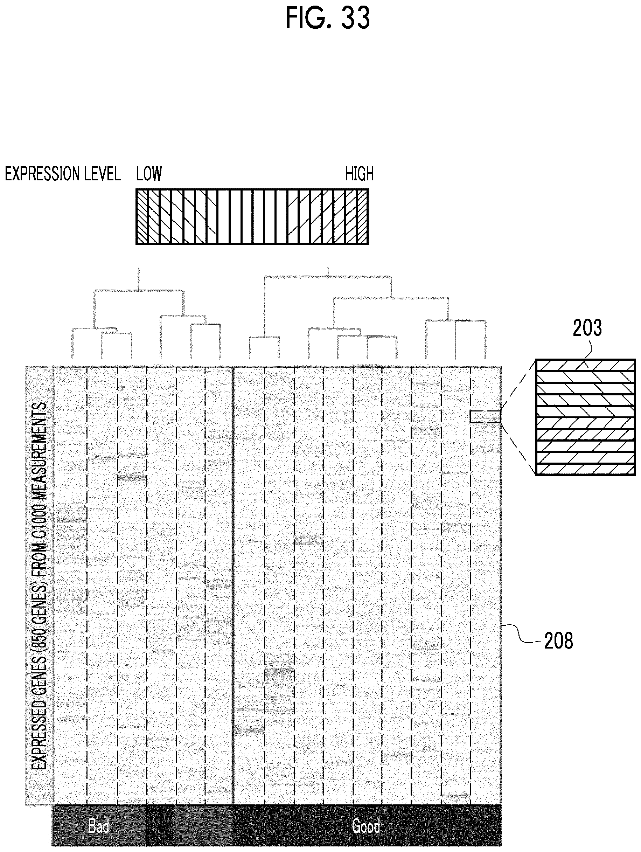

Fig. 33 shows C1000 expression level measurement result 208, obtained from the measurement carried out on the 15 samples of the iPS cell 25 before the differentiation induction. According to the measurement result 208, the samples were divided by clustering into a group of 9 samples on the right side and a group of 6 samples on the left side, and all 5 samples having a low differentiation induction efficiency, indicated by "Bad", were included in the group of 6 samples (the detection sensitivity of the sample in which the differentiation induction efficiency is low is 100%, and the specificity of the sample in which the differentiation induction efficiency is low is 83%). As a result, it was confirmed that according to C1000 according to the technology of the present disclosure, it is possible to predict the differentiation induction efficiency at the same level as the comprehensive measurement with the microarray.

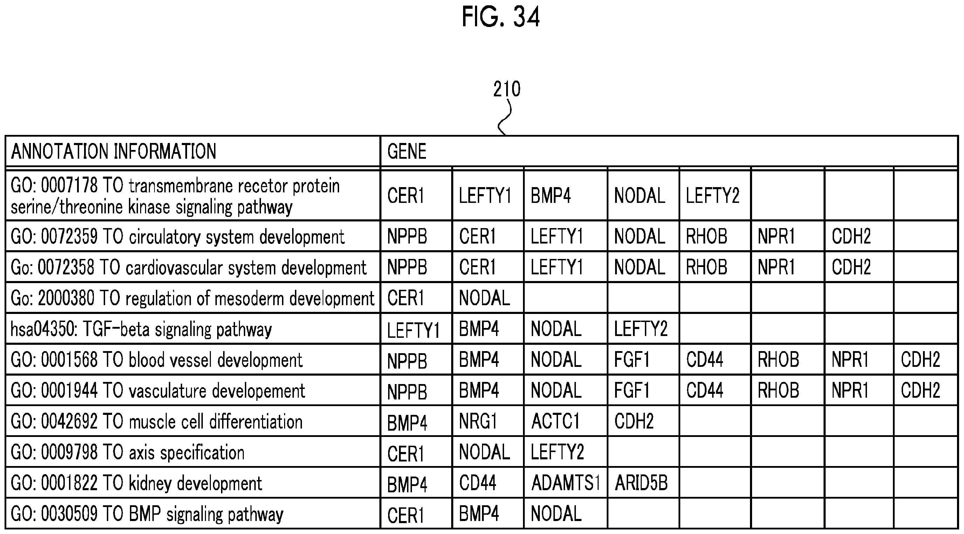

In the same manner as in the above-described analysis of the genes that had become measurement targets of the microarray, the highly expressed gene was extracted from C1000, and the high-influence annotation information was further selected. The results are shown in Table 210 of Fig. 34. According to Table 210, it can be seen that a particularly large amount of annotation information related to the exhibition of the angiogenic system function is selected. In addition, genes such as NODAL, LEFTY1, LEFTY2, CER1, and BMP4 are conspicuous, and it can be read that these genes are likely to determine the highness or lowness of the differentiation induction efficiency. That is, it was confirmed that in a case where the evaluation value is derived from the annotation information and the measurement target gene is selected based on the evaluation value as in the technology of the present disclosure, it is very useful for elucidating the characteristics of the biological specimen.

Subsequently, the analytical ability of a set of measurement genes of C1000 selected according to the technology of the present disclosure was compared with a set of measurement genes of the TaqMan scorecard on behalf of the method in the related art. The measurement results from the set of the measurement genes of the TaqMan scorecard are results simulatedly created by extracting 84 genes of the TaqMan scorecard from the results of the measurement of the comprehensive gene expression level from the microarray.

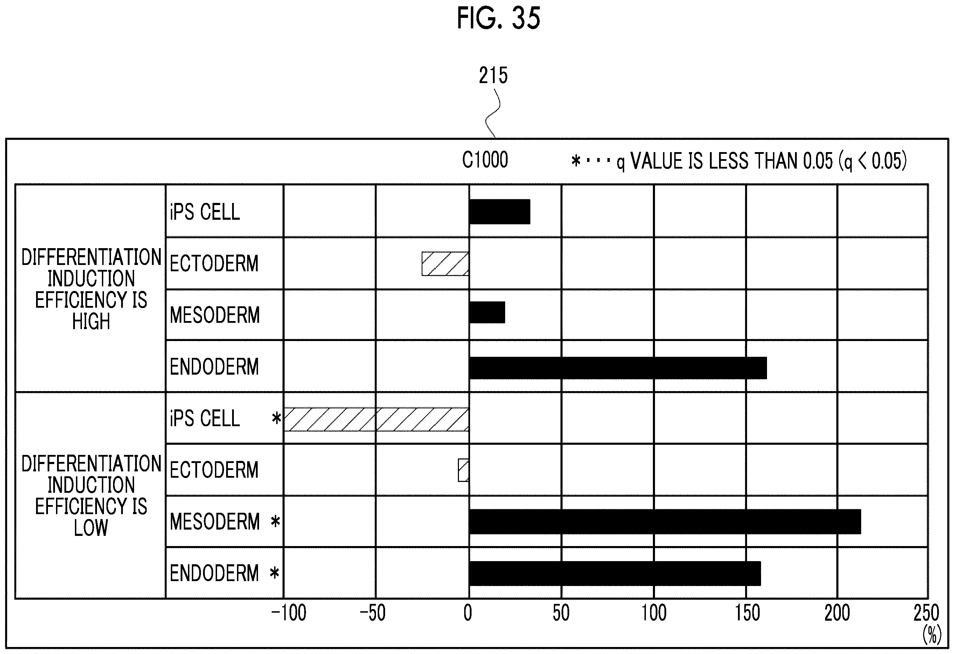

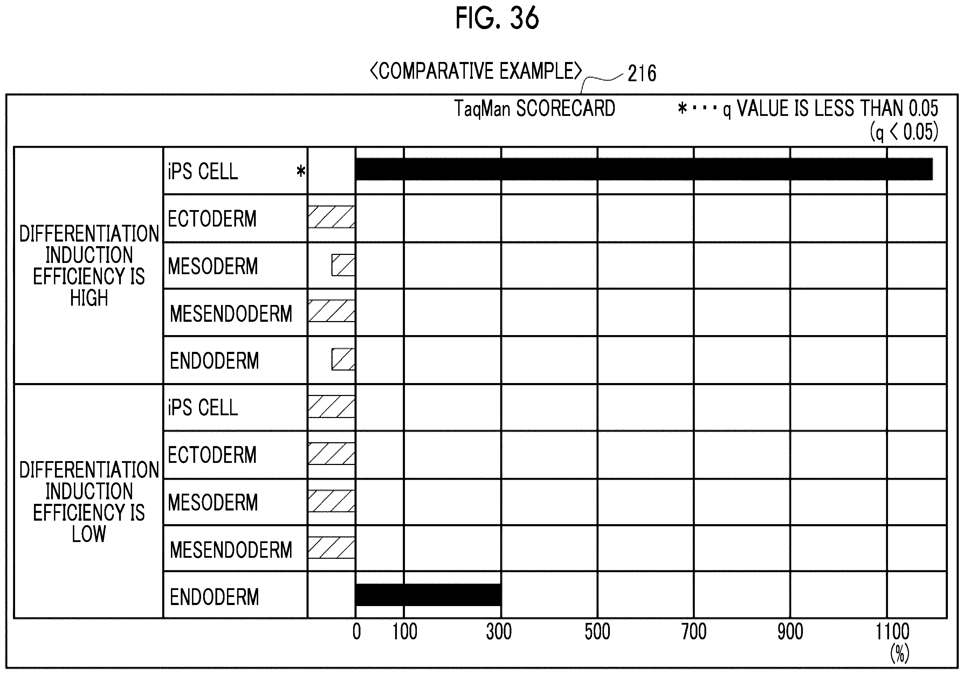

As a comparison of the analytical ability, the kind of the biological specimen in the TaqMan scorecard and the annotation information of the kind of the biological specimen in the C1000 were contrastively compared by extracting the DEGs, and the odds ratio, indicating to what extent the DEGs were enriched with respect to the gene to which each annotation information was imparted, was examined. Fig. 35 shows a bar graph 215 of the odds ratio of the set of measurement genes of C1000, and Fig. 36 shows a bar graph 216 of the odds ratio of the set of measurement genes of the TaqMan scorecard.

According to the bar graph 215 of Fig. 35, in the set of the measurement genes of C1000, the genes associated with "mesoderm" and "endoderm" were enriched in the sample having a low differentiation induction efficiency, and the number of genes associated with "iPS cell" was reduced. In addition, regarding the genes associated with the kind of each biological specimen in the case where the differentiation induction efficiency is low, the odds ratio statistically deviated significantly from 100% (the q value (the q-value) was less than 0.05 (q < 0.05)) except for "ectoderm". As a result, it was found that the set of measurement genes of C1000 has a certain analytical ability for a sample in which the differentiation induction efficiency is low. It is conceived that such results have been obtained because a sufficiently large number of measurement target genes, focusing on the kind of each biological specimen, are distributed in a well-balanced manner.

On the other hand, according to the bar graph 216 of Fig. 36, in the set of measurement genes of the TaqMan scorecard, the genes associated with "iPS cell" were enriched in the sample having a high differentiation induction efficiency, and the genes associated with "endoderm" were enriched in the sample having a low differentiation induction efficiency. However, the odds ratio statistically deviated significantly from 100% only in the genes associated with "iPS cell" in the case where the differentiation induction efficiency was high. As a result, it was found that the set of measurement genes of the TaqMan scorecard has a limited analytical ability for a sample in which the differentiation induction efficiency is low. It is conceived that such results have been obtained because, unlike the case of C1000, the number of genes distributed to each kind of biological specimen is small, and thus an extreme ratio is likely to be generated.

As described above, the technology of the present disclosure enables statistically significant elucidation even in a case where the findings are not accumulated in advance. That is, a PCR-based method, in which the test can be completed in a short time and is relatively inexpensive, can be utilized as in the case of RNA-Seq, and a wide range of applications can be expected.

### <Preparation of iPS cell-derived myocardial cell>

### [Induction of differentiation of iPS cell into myocardial cell]

15 clones of iPS cells were prepared from peripheral blood mononuclear cells derived from a plurality of donors according to the method disclosed in JP5984217B. These clones are referred to as a clone A, a clone B, a clone C, a clone D, a clone E, a clone F, a clone G, a clone H, a clone I, a clone J, a clone K, a clone L, a clone M, a clone N, and a clone O.

mTeSR 1 (registered trade name) (STEMCELL Technologies Inc.) and Matrigel (registered trade name) (Corning Inc.) were used for culturing iPS cells, and the cells were treated with a 0.5 mmol/L EDTA solution (an Invitrogen branded product manufactured by Thermo Fisher Scientific, Inc.) for 7 minutes for collection and then subcultured.

Under the above conditions, the iPS cells were subjected to the expansion culture up to two T225 flasks. The iPS cells were proliferated under the above conditions until the confluency reached about 80%, the cells were subsequently separated and detached to single cells with TrypLE Select ("TrypLE Select" is a registered trade name) (Thermo Fisher Scientific, Inc.) and collected, and the cells were suspended to 3.0 × 10⁶ cells/mL in mTeSR 1 (registered trade name) to which 1 µmol/L H1152 (FUJIFILM Wako Pure Chemical Corporation), 25 µg/ml Gentamicin (Thermo Fisher Scientific, Inc.), and 100 ng/ml bFGF (FUJIFILM Wako Pure Chemical Corporation) were added in terms of final concentration, whereby a cell suspension was obtained. Apart of this cell suspension was sampled and used for the gene expression analysis described later.

15 ml of the above cell suspension was added to a 30 ml single-use bioreactor (ABLE Corporation) and subjected to spinner culture at a rotation speed of 40 rpm. After 2 hours to 4 hours from the start of the spinner culture, the cell suspension was adjusted to a final liquid volume of 30 ml in the same culture solution and continuously subjected to the spinner culture.

1 day after the start of the spinner culture, the culture medium was replaced with a culture solution consisting of 1 µmol/L H1152, 25 µg/ml Gentamicin, 100 ng/ml bFGF, 24 ng/mL Activin A, 5% fetal bovine serum (GE Healthcare), × 0.5 mTeSR 1 (registered trade name), and × 0.5 DMEM low-glucose (Thermo Fisher Scientific, Inc.) in terms of final concentration, and then the differentiation culture was started.

Two days after the start of the spinner culture, a part of the culture solution was sampled and used for the gene expression analysis described later. In addition, a part of the culture solution was sampled to measure the number of cells, the cell concentration was prepared to 1.0 × 10⁶ cells/ml in a culture solution consisting of 25 µg/ml gentamaicin, 100 ng/ml bFGF, 24 ng/ml Activin A, 40 ng/ml BMP4, 10% fetal bovine serum, and DMEM low-glucose in terms of final concentration, and then the spinner culture was continued. From 3 days to 7 days after the start of the spinner culture, the culture medium was changed daily with the same culture solution, and then the spinner culture was continued.

Eight days after the start of the spinner culture, a part of the culture solution was sampled and used for the gene expression analysis described later. In addition, a part of the culture solution was sampled to measure the number of cells, the cell concentration was prepared to 1.0 × 10⁶ cells/ml in a culture solution consisting of 25 µg/ml gentamaicin, 16.25 µg/ml XAV939 (Sigma-Aldrich Co., LLC), 10% fetal bovine serum, and DMEM low-glucose in terms of final concentration, and then the spinner culture was continued. From 9 days to 13 days after the start of the spinner culture, the culture medium was changed every two days with the same culture solution excluding XAV939, and then the spinner culture was continued.

Fourteen days after the start of the spinner culture, a part of the culture solution was sampled, and the number of cells was measured. Table 1 shows the number of cells finally obtained after the differentiation induction.

**[Table 1]**

| Clone name | Total cell number (× 10⁶ cells) |
|---|---|
| A | 143.5 |
| B | 534.7 |
| C | 4.9 |
| D | 24.8 |
| E | 573.5 |
| F | 212.9 |
| G | 334.2 |
| H | 8.4 |
| I | 315.4 |
| J | 173.1 |
| K | 94.6 |
| L | 555.2 |
| M | 285.6 |
| N | 46.6 |
| O | 20.6 |

### [Analysis of iPS cell-derived myocardial cell aggregate by flow cytometer]

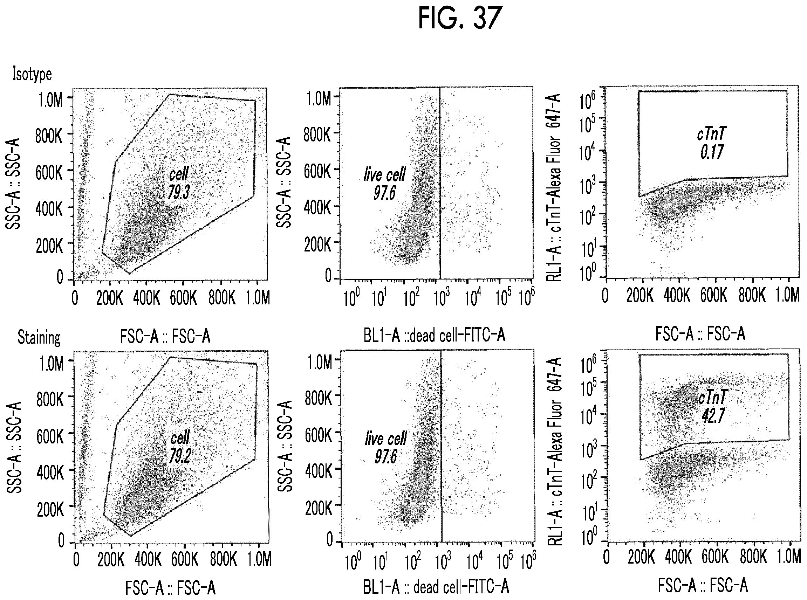

The cell aggregate 14 days after the start of the spinner culture was separated into single cells by TrypLE Select ("TrypLE Select" is a registered trade name), and then dead cells were stained with a Live/Dead (registered trade name) Fixable Green Dead Cell Stain Kit. After washing with D-PBS (Thermo Fisher Scientific, Inc.), cells were treated with formaldehyde (Sigma-Aldrich Co., LLC) at a final concentration of 4% to fix the cells. An anti-cTnT antibody (Abcam plc, ab8295) was diluted to 1/250 in D-PBS containing 0.1% Saponin (Merck Millipore) and 2% fetal bovine serum in terms of final concentration, added to 0.5 × 10⁶ cells of the fixed cells, and treated at room temperature for 1 hour. At the same time, as the isotype control, a sample to which IgG1 Isotype Control from Murine Myeloma (Sigma-Aldrich Co., LLC, M5284) diluted to 1/25 had been added was arranged in parallel. Next, an Alexa Fluor 647 ("Alexa Fluor" is a registered trade name) labeled anti-mouse IgG1 goat antibody (Thermo Fisher Scientific, Inc., A21240) was added diluted to 1/500 and treated at room temperature for 30 minutes. The labeled cells were analyzed using a flow cytometer (Thermo Fisher Scientific, Inc., Attune NxT). After gating live cells with the forward light scattering and the lateral light scattering, the cTnT positive rate (the cell number rate) was calculated from the comparison with the isotype control. A dot plot of cell aggregates induced to differentiate from the clone A is shown in Fig. 37, and the cTnT positive rate for each clone is shown in Table 2.

**[Table 2]**

| Clone name | cTnT positive rate (%) |
|---|---|
| A | 42.7 |
| B | 14.4 |
| C | 0.1 |
| D | 22.8 |
| E | 25.5 |
| F | 16.8 |
| G | 62.2 |
| H | 1.1 |
| I | 44.9 |
| J | 41.6 |
| K | 40.1 |
| L | 11.6 |
| M | 41.8 |
| N | 16.9 |
| O | 5.0 |

### <Measurement of gene expression level>

Using RNeasy Plus Mini Kit (QIAGEN N.V), respective total RNAs were extracted from the cells sampled in the culture step of the iPS cell on the 0th day, the 2nd day, and the 8th day of the spinner culture. The gene expression level of C1000 was measured using the extracted RNA as a specimen.

### <Analysis of measured genetic information>

The following analysis was carried out in order to identify a gene exhibiting the characteristics of the iPS cell used this time from the data on the gene expression level of C1000 and the cTnT positive rate which is a discrimination marker of the myocardial cell.

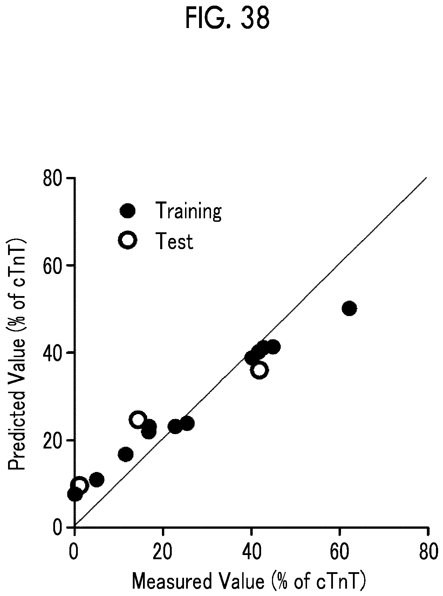

Data on the gene expression level of C1000 were acquired from the respective total RNAs acquired from the cells sampled on the 0th day, the 2nd day, and the 8th day of the spinner culture, and these were used as explanatory variables. In addition, the cTnT positive rate on the 14th day of the spinner culture was used as the response variable. A prediction model of the cTnT positive rate was created from these explanatory variables and the response variable by the regression analysis using a random forest. A prediction model was created using, as training data, evaluation datasets of 12 clones among the evaluation datasets of 15 clones, and the validity of the prediction model was verified using, as test data, the evaluation datasets of the remaining 3 clones. The results are shown in Fig. 38. Three test data were plotted, where the horizontal axis (X) is the measured value of the cTnT positive rate and the vertical axis (Y) is the predicted value of the cTnT positive rate, and as a result, each of the three plotted data was asymptotical to the straight line of Y = X, which shows the validity of the prediction model.

Table 3 shows the genes having the highest contribution in the creation of the above-described prediction model in descending order of the contributions.

**[Table 3]**

| Ranking order of degree of contribution | Gene name | Measurement time point |
|---|---|---|
| 1 | REST | Day2 |
| 2 | ALCAM | Day0 |
| 3 | sFRP2 | Day8 |
| 4 | AEN | Day8 |
| 5 | NTN1 | Day8 |
| 6 | VANGL1 | Day8 |
| 7 | CCL2 | Day0 |
| 8 | SEMA3E | Day0 |

### <Reference of gene annotation information>

In a case where the annotation information linked to each gene was referenced for the genes shown in Table 3 (that is, the biomarker indicating the characteristics of the iPS cell used this time), a document reporting that sFRP2 inhibits Wnt3a that exhibits an influence on the differentiation into the myocardial cell was found in the annotation information of sFRP2. From the content of this document, it was predicted that the efficiency of differentiation of the iPS cell into the myocardial cell is increased by adding Wnt3a to the culture solution to activate the Wnt signal pathway.

### <Improvement of culture step for inducing differentiation of iPS cell into myocardial cell>

The clone D, the clone H, and the clone O were selected from 15 clones of the iPS cells, and the same spinner culture as described above was carried out for 14 days. At that time, Wnt3a was added to the culture solution at a final concentration of 100 ng/ml on each day between the 2nd and 8th days of the culture of each clone. At the same time, a control group in which Wnt3a was not added to the culture solution was also cultured. The cell aggregates 14 days after the start of the spinner culture were analyzed by the same flow cytometry as described above, and the cTnT positive rate was examined. The results are shown in Table 4. The efficiency of differentiation of the iPS cell into the myocardial cell was significantly increased in the Wnt3a-added group as compared with the Wnt3a-free group.

**[Table 4]**

| Clone name | cTnT positive rate (%) | |
|---|---|---|
| | Wnt3a-free | Wnt3a-added |
| D | 26.8 | 42.0 |
| H | 4.64 | 24.6 |
| O | 1.58 | 9.73 |

In a case of employing the present embodiment in the production step of producing the myocardial cell from the iPS cell, it is possible to improve the productivity of the myocardial cell.

The disclosure of JP2020-106416 is incorporated in the present specification by reference in its entirety.

All publications, patent applications, and technical standards mentioned in the present specification are incorporated herein to the same extent as in a case where each individual publication, patent application, and technical standard have been specifically and individually described.

### Explanation of References

10: information processing apparatus
11: network
12: gene expression information database server (gene expression information DB server)
13: annotation information database server (annotation information DB server)
14: gene expression information database (gene expression information DB)
15: gene expression information
15E: extraction target
16: annotation information database (annotation information DB)
20: annotation information table
22, 150, 151, 158, 161, 200, 205, 206, 210: table
25: iPS cell
26: three germ layers
27: ectoderm
28: mesoderm
29: endoderm
30: tissue cell
31: crystalline lens
32: nerve cell
33: blood cell
34: bone cell
35: muscle cell
36: alveolar cell
37: intestinal cell
38: hepatocyte
45: storage device
46: memory
47 CPU (processor)
48: communication unit
49: display
50: input device
51: bus line
55 operation program (operation program of information processing apparatus)
60: instruction receiving unit
61: extraction unit
62: acquisition unit
63: derivation unit
64: selection unit
65: display control unit
70: category and number range designation information
71: prior known gene designation information
72: first distribution request
73: extraction target designation information
74: DEGs list
74G, 160G: imparted DEGs list
75: second distribution request
76: distribution information
77: evaluation value table
78: measurement target gene list
78P: temporary measurement target gene list
80: category designation screen
81, 96: pull-down menu
82 to 84, 106: input box
85, 97, 107: add button
86, 98, 108: designation button
87, 121Ato 121D: display region
88, 91: message
90: warning screen
92: OK button
95: prior known gene designation screen
105: extraction target designation screen
115: selection order table group
116A to 116D: selection order table
120: measurement target gene display screen
122: save button
123: print button
124: confirm button
202: measurement results of gene expression level from microarray
203: bar
208: C1000 expression level measurement result
215, 216: bar graph
ST100, ST110, ST120, ST130, ST140, ST150, ST160, ST170, ST180, ST190, ST210, ST230, ST250: step
ST200: step (acquisition processing)
ST220: step (derivation processing)
ST240: step (selection processing)

## Claims

1. A biomarker identifying method that is a method of identifying a biomarker indicating characteristics of a cell A that is used for producing a cell B, the biomarker identifying method comprising the following (1) to (4):
(1) deriving an evaluation value for each of a plurality of biomarkers based on annotation information imparted to each of the plurality of biomarkers and selecting a measurement target biomarker from among the plurality of biomarkers based on the evaluation value;
(2) acquiring evaluation data of the measurement target biomarker from at least one of the cell A or a culture system, at least before start of culture of the cell A or during the culture;
(3) acquiring evaluation data of a discrimination marker of the B cell from at least one of the cell A or the culture system, at least at a final stage of the culture of the cell A or after end of the culture; and
(4) identifying at least one biomarker indicating characteristics of the cell A that is used for producing the cell B from among the measurement target biomarkers, based on the evaluation data of the measurement target biomarker and the evaluation data of the discrimination marker of the cell B.

2. The biomarker identifying method according to claim 1,
wherein the biomarker includes at least one selected from the group consisting of an expression level of a gene, an expression level of a protein, and a generation amount of a metabolite.

3. The biomarker identifying method according to claim 1 or 2,
wherein the (2) is carried out at a plurality of time points.

4. The biomarker identifying method according to any one of claims 1 to 3,
wherein the cell A is a pluripotent stem cell and the cell B is a differentiated cell.

5. A cell producing method that is a method of culturing a cell A to produce a cell B, the cell producing method comprising the following (A) to (C):
(A) carrying out the biomarker identifying method according to any one of claims 1 to 3 to identify at least one biomarker indicating characteristics of the cell A;
(B) identifying at least one of a signal transduction in which the biomarker is involved or a mechanism by which the biomarker varies with reference to annotation information of the identified biomarker; and
(C) culturing the cell A under culture conditions, in which at least one of the signal transduction or the mechanism is inhibited or promoted, to produce the cell B.

6. The cell producing method according to claim 5,
wherein the cell A is a pluripotent stem cell and the cell B is a differentiated cell.
